(19) Europäisches Patentamt
European Patent Office
Office européen des brevets

(11) Publication number : 0 311 619 B1

(12) EUROPEAN PATENT SPECIFICATION

(45) Date of publication of patent specification : 06.03.91 Bulletin 91/10

(51) Int. Cl.$^5$ : C07C 47/02, C07C 45/50, C07F 9/50, B01J 31/24

(21) Application number : 87903989.9

(22) Date of filing : 12.06.87

(86) International application number : PCT/US87/01362

(87) International publication number : WO 87/07600 17.12.87 Gazette 87/28

# (54) CHELATE LIGANDS FOR LOW PRESSURE HYDROFORMYLATION CATALYST AND PROCESS EMPLOYING SAME.

(30) Priority : 13.06.86 US 873918
18.02.87 US 16154
18.02.87 US 16155

(43) Date of publication of application : 19.04.89 Bulletin 89/16

(45) Publication of the grant of the patent : 06.03.91 Bulletin 91/10

(84) Designated Contracting States : DE GB SE

(56) References cited :
FR-A-24 192 68
GB-A- 2 014 138
Chemical Abstracts, volume 91, 1979, Columbus, Ohio, US, see p. 792, abstract 91764v, & JP-A-7939059

(73) Proprietor : EASTMAN KODAK COMPANY (a New Jersey corporation)
343 State Street
Rochester New York 14650 (US)

(72) Inventor : DEVON, Thomas, James
109 Katy Drive, Rt. 7
Longview, TX 75601 (US)
Inventor : PHILLIPS, Gerald, Wayne
1519-B North Eastman Road Apt. B
Longview, TX 75601 (US)
Inventor : PUCKETTE, Thomas, Allen
306 Jamie Court
Longview, TX 75601 (US)
Inventor : STAVINOHA, Jerome, Leonard, Jr.
204 Terese Street
Longview, TX 75601 (US)
Inventor : VANDERBILT, Jeffrey, James
2403 Woodhollow Court
Longview, TX 75604 (US)

(74) Representative : Parent, Yves et al
Kodak-Pathé Département Brevets et Licences Centre de Recherches et de Technologie Zone Industrielle
F-71102 Chalon-sur-Saône Cédex (FR)



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention concerns novel chelate ligands and hydroformylation catalysts and processes employing the same wherein one or more olefins and/or non-conjugated diolefins and/or other unsaturated organic compounds, all hereinafter referred to as hydroformylation stock, may be converted to aldehydes for use as such or for conversion by known methods, to products such as alcohols and acids. More particularly, the invention concerns ligands especially useful for oxo or hydroformylation processes designed for relatively low pressure operation for the preparation of unusually high proportions of normal or unbranched aldehydes from α-olefins, particularly n-butyraldehyde from propylene.

The chelate ligands contemplated by the present invention are compounds of the general formula

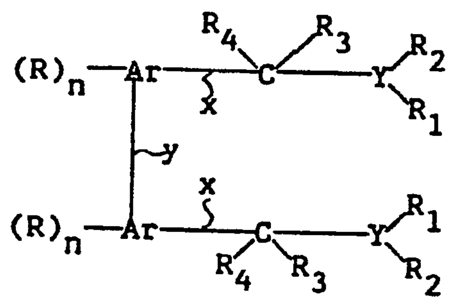

wherein :

each Ar is independently selected from aromatic ring compounds having 6 up to 14 carbon atoms, e.g., phenyl, naphthyl, phenanthryl and anthracenyl ;

the x bonds and the y bonds are attached to adjacent carbon atoms on the ring structures ;

each R, when present as a substituent, is independently selected from alkyl, alkoxy, aryloxy, aryl, aralkyl, alkaryl, alkoxyalkyl, cycloaliphatic, halogen (except C1, Br or I in the ortho-position), alkanoyl, alkanoyloxy, alkoxycarbonyl, or formyl ;

n is a whole number in the range of 0-4 were Ar is phenyl ; 0-6 where Ar is naphthyl ; and 0-8 where Ar is phenanthryl or anthracenyl ;

each $R_1$ and $R_2$ is independently selected from alkyl, aryl, aralkyl, alkaryl or cycloaliphatic ;

each $R_3$ and $R_4$ is independently selected from hydrogen and the R substituents ;

each of the above alkyl groups or moieties is straight or branched chain of 1-20 carbons, preferably 1-8 carbons ;

each aryl group contains 6-10 ring carbons ; each cycloaliphatic group contains from 4-8 ring carbons; and

each Y is independently selected from the elements N, P, As, Sb and Bi, with P being preferred.

In a particular embodiment of the present invention, the ligands employed in the practice of the present invention are compounds of the formula

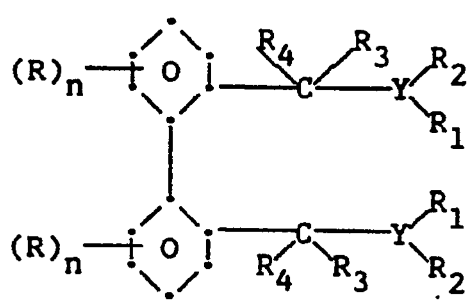

wherein

n is 0-4 ;

each R is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each of the above alkyl groups or moieties is straight or branched chain of 1-20 carbons, each aryl group contains 6-10 ring carbons, and each cycloaliphatic group contains from 4-8 ring carbons ; and

each Y is independently selected from the elements N, P, As, Sb and Bi, with P being preferred.

In another particular embodiment of the present invention, the ligands contemplated are compounds of the general formula

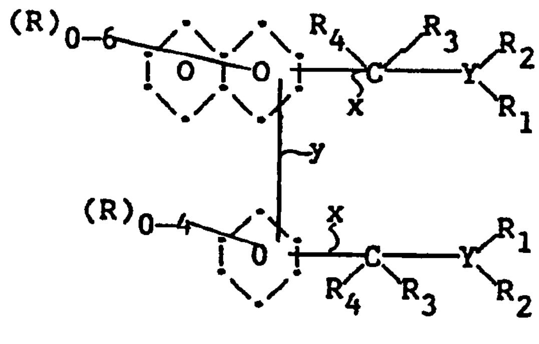

wherein :

the x bonds and the y bonds are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each of the above alkyl groups or moieties is straight or branched chain of 1-20 carbons, preferably 1-8 carbons, each aryl group contains 6-10 ring carbons, and each cycloaliphatic group contains from 4-8 ring carbons ; and

each Y is independently selected from the elements N, P, As, Sb and Bi, with P being preferred.

In yet another particular embodiment of the present invention, the ligands contemplated are compounds of the general formula

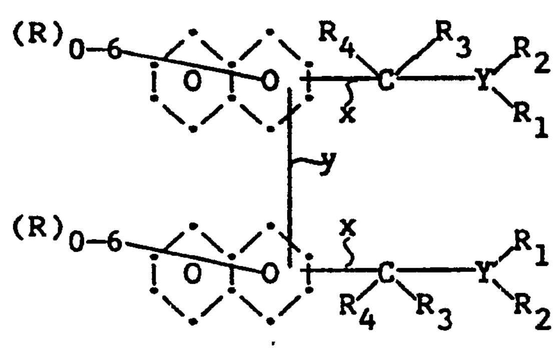

wherein

the x bonds and the y bonds are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the R substituents ;

each of the above alkyl groups or moieties is straight or branched chain of 1-20 carbons, preferably 1-8 carbons, each aryl group contains 6-10 ring carbons, and each cycloaliphatic group contains from 4-8 ring carbons ; and

each Y is independently selected from the elements N, P, As, Sb and Bi, with P being preferred.

Preferred of the present ligands are 2,2′-bis-(diphenylphosphinomethyl)-1,1′-biphenyl ; 2,2′-bis-(dibenzylphosphinomethyl)-1,1′-biphenyl ; 2,2′-bis-(phenylbenzylphosphinomethyl)-1,1′-biphenyl ; 2,2′-

bis(diisobutylphosphinomethyl)-1,1′-biphenyl ; 2-(diphenylphosphinomethyl)-1-[2-(diphenylphosphinomethyl) phenyl]naphthalene ; and 2,2′-bis(diphenylphosphinomethyl)-1,1′-binaphthyl.

The present hydroformylation process in its broad sense comprises contacting at least one olefin having from 2 to 20 carbon atoms in a reaction zone at a temperature of from 20°C to 250°C and a pressure of from 103 Kpascals (15 psig) to 5517 Kpascals (800 psig) with syn gas ($H_2$, CO) and a catalyst comprising rhodium in chemical complex with one or more of the above ligands for a sufficient period of time to permit reaction of said olefin with said syn gas to form aldehyde product.

Alpha-olefins with heteroatom substitution on the molecule such as allyl alcohol, allyl acetate, 4-hydroxybutene-1, and the like may also be used in this invention. Also, branched olefins such as isobutene and internal olefins such as cis-butene-2 may be used herein as feedstocks for the preparation of aldehyde products. Diolefins such as 1,7-octadiene and the like may also be used to prepare dialdehyde products provided that the two carbon-carbon double bonds are not in conjugation.

The present ligands, in particular, the 2,2′-bis(phosphinomethyl)-1,1′-biphenyl ligand class (hereinafter BISBI), the 2-(diphenylphosphinomethyl)-1-[2-(diphenylphosphinomethyl)phenyl]-naphthalene ligand class (hereinafter, PHENAP), the 2,2′-bis(diphenylphosphinomethyl)-1,1′-binaphthyl ligand class (hereinafter, NAPHOS) have great utility as bidentate ligand modifiers for the low pressure rhodium hydroformylation of alphaolefins to prepare aldehyde products with unusually high ratios of normal to branched isomers. This invention effects the efficient use of olefinic feedstocks to prepare desirable linear aldehyde products in high yield. Such products from propylene include n-butyraldehyde which is used to prepare the commercial solvent n-butanol. The hydroformylation of 1-butene and 1-pentene yield intermediate aldehyde products useful for the preparation of the solvents 1-pentanol and 1-hexanol, respectively. The hydroformylations of 1-hexene and 1-octene yield aldehyde products used to prepare the commercially valuable carboxylic acids, n-heptanoic acid and n-nonanoic acid. These same aldehyde products may be converted into alcohols useful for the preparation of plasticizers, synthetic lubricants, and detergents. Likewise, the hydroformylation of higher olefins such as 1-decene and 1-dodecene yield aldehyde precursors to 1-undecanol and 1-hydroxytridecane useful as fabric softeners and ingredients in plasticizers and detergents. These ligands show improvements in hydroformylation technology in one or more areas such as high normal to iso ratios employing relatively small amounts of ligand, effective in low pressure systems, increased catalytic activity and retention thereof over extended periods, and increased catalyst stability. The ligands of the invention are particularly useful in their unique ability to produce the desired high normal/iso ratios even at desirable low levels of ligand.

As a general statement of the actual chemical composition of the present active catalyst species in the reaction zone, the species preferably comprises rhodium complexed with (a) a ligand defined by the above structural formulae in a molar ratio of ligand/Rh of 1/1, (b) H in an atomic ratio of H/Rh of 1/1, and (c) carbon monoxide in a molar ratio of CO/Rh of 2/1.

The present process is carried out preferably in a gas sparged reactor such that the catalyst which is dissolved in a high boiling organic solvent under pressure does not leave the reaction zone with the aldehyde product which is taken overhead by the unreacted gases. The overhead gases are then chilled in a vapor liquid separator to condense out the aldehyde product, the gases being recycled to the reactor and the liquid product let down to atmospheric pressure for separation and purification by conventional techniques. A side draw from the reactor preferably is provided so that a small amount of the catalyst can be withdrawn at a desirable rate for more complete distillation and/or regeneration and returned to the reactor after the addition of make-up ligand thereto.

The metal catalyst components are charged preferably with solvent to the reactor through suitable pressurized pumping means, preferably in their soluble forms, e.g., their carboxylate salts or mineral acid salts or the like well known to the art as disclosed, for example, in U.S. Patent 2,880,241. Charged therewith or separately is one or more of the present modifying ligands in amounts such that the molar ratio of ligand to rhodium in the reactor is from 1.0 to 200 or more, preferably from 2.0 to 10.0, and most preferably from 2.3 to 4.0

The process is particularly effective at pressures from 103-5517 Kpascals (15 to 800 psig) with from 690 to 2760 Kpascals (100 to 400 psig) being preferred, and from 1655 to 1931 Kpascals (240 to 280 psig) being most preferred. The reaction temperatures can vary from 20 to 250°C, but preferably from 50 to 175°C and most preferably from 80 to 150°C. In a highly preferred embodiment, the above process is carried out wherein at steady state hydroformylation conditions in said reaction zone the ratio of Rh(mg.)/solvent (ml) is from 0.07 to 0.28, the ratio of [olefin feed in liters (STP)/min]/mg. of Rh is from 0.03 to 0.30, the ratio of [CO or $H_2$ feed in liters (STP)/min]/mg. of Rh is from 0.015 to 1.5, the temperature is maintained at from 80°C to 150°C, and the reactor pressure is maintained at from 1655 to 1931 Kpascals (240 psig to 280 psig). The term "STP" refers to standard temperature of 273°K and standard pressure of 101,3 Kpascals (760 mm

Hg).

In the process, the syn gas is introduced into the reactor in a continuous manner by means, for example, of a primary compressor, and the ratio of hydrogen to carbon monoxide in the feed may be selected according to the particular olefin being hydroformylated and the reaction conditions present, as is well known in the art. Generally, the molar ratio of hydrogen to carbon monoxide in the reactor is maintained within the range of 0.5 to 4.0, but it has been found in many hydroformylations that the rate of reaction as well as yield of the desired product may be increased by increasing the hydrogen to carbon monoxide molar ratio above 4.0, and up to 10.0 or more. In the reactor zone the syn gas preferably is present in a molar excess (total moles of $H_2$ + CO) with respect to the olefin and the molar ratio varies typically from 0.25 to 20, preferably from 1.2 to 6. In a liquid overflow reactor, the above molar ratio may have a lower limit of 0.02.

The olefin is fed to the reactor by means of suitable pumps capable of operating under substantial pressures, and the feed rates of the olefin and syn gas are selected to maintain the above-recited molar ratios of these reactants in the reactor. Typical olefins to which the present invention is applicable include straight or branched chain α-olefins containing from 2 to 20 carbon atoms and preferably from 2 to 10 carbon atoms, and optionally containing groups or substituents which do not interfere with the hydroformylation process. Illustrative of such α-olefins are ethylene, propylene, 1-butene, 2-methylpropylene, 2-methyl-1-butene, 1-pentene, 1-hexene, 1-heptene, 1-octene, 2-ethyl-1-hexene, 1-dodecene, 1-octadecene and allyl alcohol. Also useful with the present invention are the internal olefins such as butene-2 and cyclic olefins such as cyclooctene. If desired, mixtures of olefins, particularly ethylene and propylene, can be fed to the reactor.

Any suitable solvent which does not adversely affect the hydroformylation process and which is inert with respect to the catalyst, olefin feed, syn gas and the hydroformylation products may be used. Inert solvents of this nature are well known to those skilled in the art and include benzene, xylene, toluene and their substituted derivatives, pentanes, naphtha, kerosene, mineral oils, cyclohexane, cyclopentane, ethers, esters, etheresters, alcohols, acetals, ketones, and various mixtures thereof. Preferred solvents are those which are sufficiently high boiling to remain for the most part in the gas sparged reactor, and include 2,2,4-trimethyl-1,3-pentanediol monoisobutyrate (TMPDMI ; available from Eastman Chemicals Division of Eastman Kodak as Texanol® solvent), and its isomers, and the by-products such as alcohols, esters, acetals, and hydroxyaldehydes produced in the hydroformylation reaction and retained as high boiling liquids at the bottom of the subsequent distillation columns.

Aldehyde product also may be prepared batchwise with the present invention by contacting the olefin, hydrogen, and carbon monoxide with the present catalyst in an autoclave. High boiling aldehyde products such as normal nonanal may be prepared in a continuous manner with the aldehyde product being removed from the reactor zone as a liquid in combination with the catalyst. The aldehyde product may be separated from the catalyst by conventional means such as by distillation, and the catalyst then recycled back to the reactor. It will be apparent to those skilled in the art that other reactor schemes may be used with this invention.

The present process can be carried out with very small amounts of catalyst containing from $1 \times 10^{-6}$ moles of rhodium (calculated as Rh°) per mole of olefin in the reactor zone. However, such low catalyst concentrations are not commercially desirable since the reaction rates are low. The upper catalyst concentration is essentially unlimited and appears to be dictated principally by the high cost of rhodium and the fact that no advantage is evident in the use of catalyst containing above $1 \times 10^{-1}$ moles of rhodium per mole of olefin in the reactor zone. A concentration of from $1 \times 10^{-5}$ moles to $1 \times 10^{-2}$ moles of rhodium per mole of olefin is preferred, and from $1 \times 10^{-4}$ to $5 \times 10^{-2}$ is most preferred.

This invention will be illustrated further by the following examples although it will be understood that these examples do not limit the invention and are for purposes of illustration only.

## LIGAND PREPARATION

The synthesis of the present chelating diphosphine ligands is straightforward thus making them easily prepared in practical quantities. Below are given some of the synthetic routes available to prepare the ligands, the phosphorylation of 2,2′-bis-(bromomethyl)-1,1′-biphenyl by alkali metal "M" phosphine anions being a particularly useful synthesis. Examples are given herein of the synthesis of the bis(diphenylphosphino), bis(phenylbenzylphosphino), and bis(diisobutylphosphino) derivatives using this method (Reaction I). Likewise, the diphosphino chelate ligands may be prepared by the oxyphosphorylation of the above dibromo compound by its reaction with alkali metal salts of the phosphine oxide anion and subsequent reduction to the diphosphine chelating ligand (Reaction II) by any of a number of reducing agents such as lithium aluminum hydride.

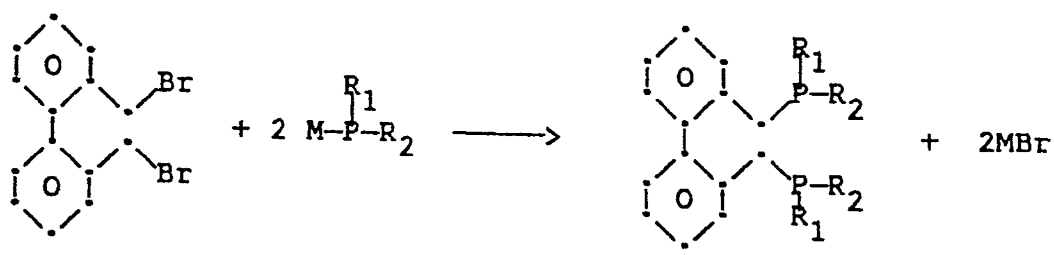

Reaction I

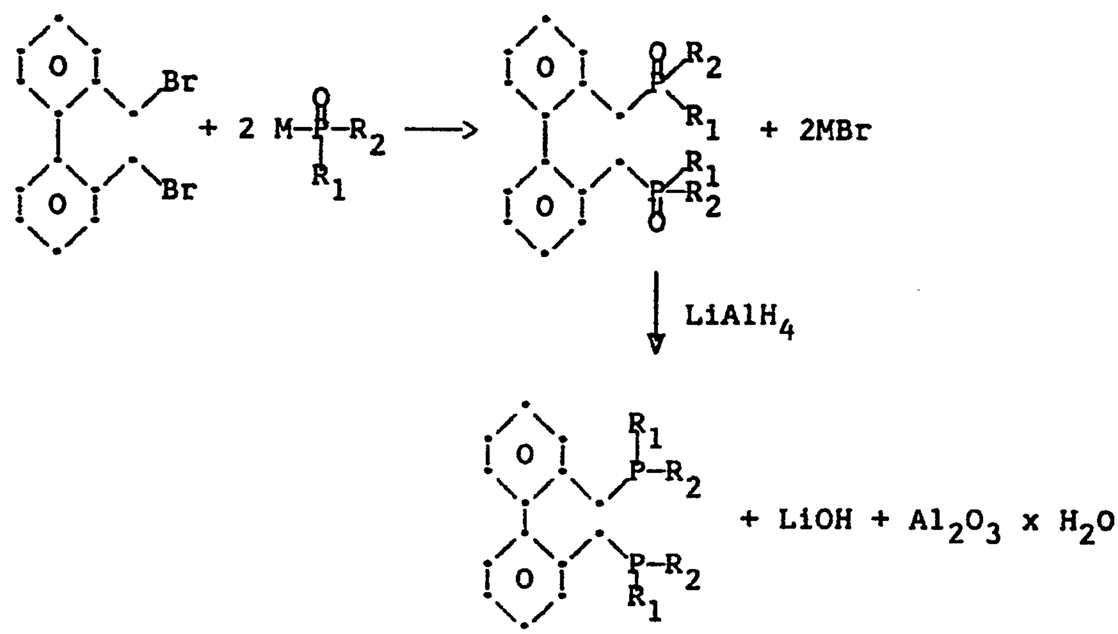


Reaction II

The above 2,2′-bis(bromomethyl)-1,1′-biphenyl intermediate is prepared in high yield from 2,2′-bis(hydroxymethyl)-1,1′-biphenyl by its reaction with $PBr_3$. The diol precursor is easily obtained in known manner by either standard catalytic hydrogenation or by lithium aluminum hydride reduction of diphenic acid. The diol is also easily prepared by reduction of 2,2′-bis(formyl)-1,1-biphenyl, obtained by the high yield ozonolysis of phenanthrene in known manner.

The diphosphine ligand 2-(diphenylphosphinomethyl)-1-[2-(diphenylphosphinomethyl)phenyl]-naphthalene (PHENAP) was prepared by a reaction sequence shown below. In this sequence 2-methyl-1-(2-methylphenyl)naphthalene (I) was synthesized by the nickel-catalyzed cross-coupling of 2-bromotoluene and the Grignard reagent produced from 1-bromo-2-methylnaphthalene by the Maigrot/Mazaleyrat procedure published in Synthesis, 317 (1985). The resulting biaryl was brominated using N-bromosuccinimide, with N,N′-azobis(isobutyronitrile) as catalyst, to form 2-bromomethyl-1-[2-(bromomethyl)phenyl]naphthalene (II) by the Bestman/Both procedure, Chem. Ber., 107, 2926, (1974). The dibromide was then treated with methyl diphenylphospinite by the procedure of Arbuzov/Nikonorov Zhur Obshchei Khim., 18, 2008 (1948) ; Chem. Abstracts, 43, 380li (1949) to give the bisphosphine dioxide (III). Reduction of the dioxide with trichlorosilane/triethylamine by the procedure of Fritzsche/Hasserodt Chem. Ber., 98, 171 (1965) produced the desired product PHENAP.

Reaction Sequence For PHENAP

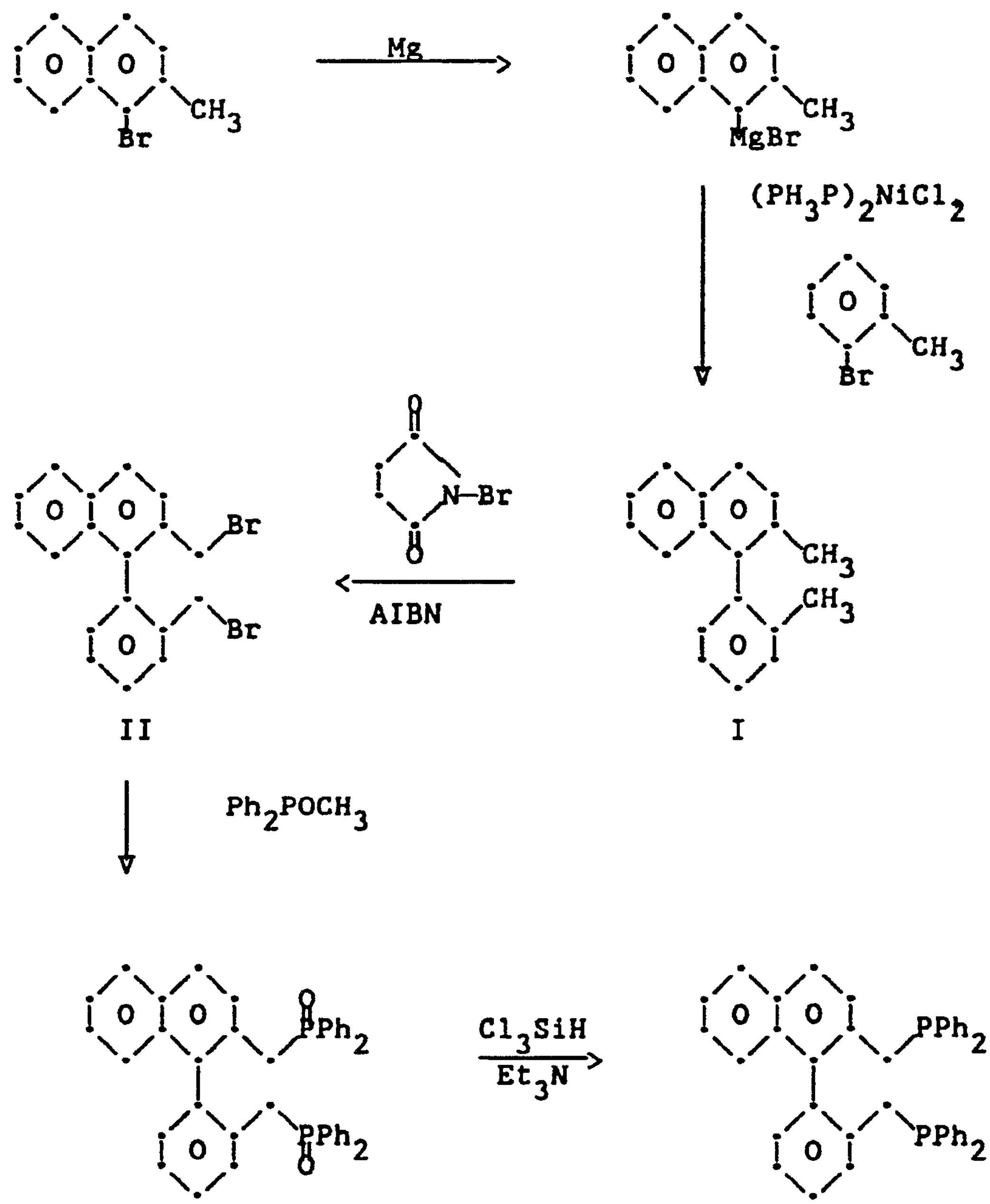


II, I, III, PHENAP

wherein Ph = phenyl.

NAPHOS was synthesized by a sequence of published procedures. The 2,2′-dimethyl-1,1′-binaphthyl was prepared from 1-bromo-2-methyl-naphthalene by the method of Maigrot and Mazaleyrat, Synthesis, 317 (1985). Benzylic bromination of 2,2′-dimethyl-1,1′-binaphthyl with N-bromosuccinimide and catalytic N,N′-azobis(isobutyronitrile), by the procedure of Bestmann and Both Chem. Ber., 107, 2926 (1974), afforded 2,2′-bis(bromomethyl)-1,1′-binaphthyl. Using methods outlined by Tamao, et al in Tetrahedron Letters No. 16, pp 1389-1392, 1977, Pergamon Press, Gr. Brit., the dibromide was converted to NAPHOS (1,1) dioxide by reaction with methyl diphenylphosphinite and the dioxide was reduced with trichlorsilane/triethylamine to yield NAPHOS. It is noted that none of these references are concerned in any way with the use of NAPHOS for oxo or hydroformylation reactions.

All experimental procedures involving phosphines or organometallic compounds were run under an atmosphere of nitrogen using dry, deoxygenated solvents. Tetrahydrofuran (THF) was distilled under nitrogen from sodium/benzophenone ketyl. Chemical shifts for nuclear magnetic resonance (NMR) spectra

are reported in parts per million (δ) downfield from tetramethylsilane for $^1H$ NMR spectra and relative to aqueous $H_3PO_4$ for $^{31}P$ NMR spectra.

Example 1 - 2,2'-Bis(hydroxymethyl)-1,1'-biphenyl

Lithium aluminum hydride (12.60 grams, 0.332 mol) and THF (175 ml) were placed in a dry 500 ml three-necked round-bottomed flask fitted with a condenser, addition funnel, nitrogen inlet, and magnetic stirrer. The mixture was cooled with an ice bath and diphenic acid (40.00 grams, 0.165 mol) in THF (100 ml) was added dropwise to the stirring mixture. After the addition was complete, the flask was removed from the ice bath and allowed to warm to room temperature. The reaction mixture was heated at reflux for 2 hours, then stirred overnight at room temperature. After cooling the mixture with an ice bath, water (12.6 ml) was added dropwise, followed by the successive dropwise addition of 15% aqueous sodium hydroxide (12.6 ml) and water (38 ml). The resulting yellow mixture was warmed to room temperature, and the solids were separated by vacuum filtration. The filtrate was placed on a rotary evaporator to remove the solvent. The remaining brownish-yellow solid was recrystallized from toluene-hexane to give 28.60 grams (81% yield) of light brown solid product, melting point 105° to 108°C. $^1H$ NMR ($CDCl_3$) : δ 2.77 (br s, 2H, –OH) ; 3.95 (s, 4H, $-CH_2-$); 6.50-7.25 (m, 8H, aromatic).

Example 2 - 2,2'-Bis(bromomethyl)-1,1'-biphenyl

2,2'-Bis(hydroxymethyl)-1,1'-biphenyl (25.00 grams, 0.117 mol) and methylene chloride (200 ml) were placed in a 500 ml round-bottomed flask equipped with a magnetic stirrer and an addition funnel with a $CaCl_2$ drying tube. The stirred mixture was cooled with an ice bath, and phosphorus tribromide (23.1 ml, 66.50 grams, 0.246 mol) was added dropwise from the addition funnel. After the addition was complete, the reaction mixture was removed from the ice bath and stirred overnight at room temperature. The mixture was again cooled with an ice bath, and water (35 ml) was added slowly. After stirring for 1 hour, additional water (75 ml) was added. The layers were separated in a separatory funnel, and the aqueous layer extracted twice with $CH_2Cl_2$. The combined organic layer was washed with saturated aqueous $NaHCO_3$ and water and was then dried with $MgSO_4$. The solvent was removed on a rotary evaporator to give 37.05 grams (93% yield) of light yellow solid which was suitable for use without further purification. The melting point was 85°C to 88°C. $^1H$ NMR ($CDCl_3$) : δ 4.10 (d, J = 10 Hz, 2H, –CH–Br) ; 4.22 (d, J = 10 Hz, 2H, –CH–Br) ; 6.92-7.53 (m, 8H, aromatic)

Example 3 - 2,2'-Bis(diphenylphosphinomethyl)-1,1'-biphenyl

Diphenylphosphine (10.0 ml, 10.70 grams, 0.057 mol) was dissolved in dry THF (115 ml) under nitrogen in a 300-ml three-necked round-bottomed flask equipped with a magnetic stirrer, addition funnel, and condenser with a nitrogen inlet. The solution was cooled to about –70°C with a dry ice/acetone bath and n-butyllithium (35.9 ml of a 1.6 M solution in hexane, 0.057 mol) was added dropwise from the addition funnel. The orange solution was stirred 1 hour in the cold bath. A solution of 2,2'-bis(bromomethyl)-1,1'-biphenyl (9.30 grams, 0.027 mol) in THF (50 ml) was added dropwise from the addition funnel over about 20 minutes. The solution was allowed to stir overnight at room temperature and was then heated at reflux for 3 hours. Saturated aqueous $NH_4Cl$ (about 50 ml) was added to the stirring solution at room temperature. Diethyl ether (75 ml) was added, and the layers were separated in a separatory funnel. The aqueous layer was extracted twice with diethyl ether. The combined organic solution was washed twice with water. The organic solvent was evaporated on a steam bath under a stream of nitrogen to give a thick, oily residue. The residue was recrystallized from ethanol/diethyl ether to give 10.64 grams (71% yield) of white solid, melting point 84° to 87°C. $^1H$ NMR ($CDCl_3$) : δ 3.15 (s, 4H, –CH2–) ; 6.60-7.40 (m, 28H, aromatic). $^{31}P$ NMR ($CDCl_3$) : δ + 9.

Example 4 - 2,2'-Bis(diisobutylphosphinomethyl)-1,1'-biphenyl

Diisobutylphosphine (1.66 grams, 11.37 mmol) was dissolved in THF (25 ml) under nitrogen and cooled with a dry ice/acetone bath. n-Butyllithium (7.11 ml of a 1.6 M solution in hexane, 11.37 mmol) was added dropwise to the stirring solution which was then allowed to stir for 1 hour in the cold bath. 2,2'-Bis(bromomethyl)-1,1'-biphenyl (1.89 grams, 5.55 mmol) in THF (10 ml) was added dropwise at –70°C. The solution was allowed to stir overnight at room temperature and was then heated at reflux for 1 hour. Saturated aqueous $NH_4Cl$ was added to the solution at room temperature. Diethyl ether was added and the layers were separated in a separatory funnel. The aqueous layer was extracted twice with diethyl ether, and the

combined organic solution was then washed twice with water. The solvent was removed on a steam bath under a stream of nitrogen. The oily residue was then placed on a Kugelrohr distillation apparatus to remove low boiling material at about 175°C and (1 mm Hg) 1,33 mbar leaving 1.88 grams (72% yield) of a thick orange glassy product. $^1$H NMR ($CDCl_3$) : δ 0.65-1.67 (complex, 36H, aliphatic) ; 2.42 (s, 4H, benzylic) ; 6.67-7.30 (complex, 8H aromatic). $^{31}$P NMR ($CDCl_3$) : δ + 31.

Example 5 - 2,2′-Bis(dibenzylphosphinomethyl)-1,1′-biphenyl Dioxide

Dibenzylphosphine oxide (6.93 grams, 30.1 mmol) and THF (100 ml) were placed in a 300-ml three-necked flask and cooled at –40°C under nitrogen. n-Butyllithium (18.84 ml of a 1.6 M solution in hexane, 30.1 mmol) was added dropwise from an addition funnel over about 10 minutes and the resulting yellow solution was stirred for 1 hour at –30°C to –35°C. 2,2′-Bis-(bromomethyl)-1,1′-biphenyl (5.00 grams, 14.7 mmol) in THF (20 ml) was added dropwise to the cold solution. When the addition was complete, the solution was warmed to room temperature and was then heated at reflux for 1.5 hours. Saturated aqueous $NH_4Cl$ was added and the layers were separated. The aqueous layer was extracted twice with diethyl ether. The combined organic solution was washed with saturated aqueous NaCl. The organic solvent was evaporated on a steam bath under a stream of nitrogen to give a light brown solid. The product was recrystallized from acetone to give a first crop of 3.57 grams (38% yield) of white solid, melting point 203° to 205°C. No attempt was made to recover a second crop. $^1$H NMR ($CDCl_3$) : δ 2.07-3.08 (complex, 12H, benzylic) ; 6.57-7.47 (complex, 28H, aromatic). $^{31}$P NMR ($CDCl_3$) : δ -43.

Example 6 - 2,2′-Bis(dibenzylphosphinomethyl)-1,1′-biphenyl

Chlorotrimethylsilane (4.1 ml, 32.2 mmol) was added to lithium aluminum hydride (1.22 grams, 32.2 mmol) in THF (20 ml) at –72°C. The mixture was removed from the cold bath, stirred 2 hours, and then cooled again at –35°C. A suspension of the above 2,2′-bis(dibenzylphosphinomethyl)-1,1′-biphenyl dioxide (3.40 grams, 5.32 mmol) in THF (45 ml) was added by cannula. The mixture was stirred 0.5 hour at –30°C, then overnight at room temperature. The reaction mixture was cooled in an ice bath and quenched by the successive, dropwise addition of water (1.2 ml), 15% aqueous NaOH (1.2 ml) and water (3.6 ml). The resulting mixture was filtered, and the solid was washed with diethyl ether. The filtrate was evaporated on the steam bath under a stream of nitrogen. The residual solid was heated in ethanol, then cooled and filtered to give 2.00 grams (62% yield) of white solid, melting point 163° to 167°C.

$^1$H NMR ($CDCl_3$) : δ 2.43 (s, 12H, benzylic) ; 6.50-7.17 (complex, 28H, aromatic). $^{31}$P NMR ($CDCl_3$) : δ + 9.5.

Example 7 - 2,2′-Bis(benzylphenylphosphinomethyl)-1,1′-biphenyl

Benzyldiphenylphosphine (9.74 grams, 35.3 mmol) was dissolved in THF (100 ml) under nitrogen in a 250 ml three-necked round-bottomed flask equipped with a magnetic stirrer, thermometer, and condenser with a nitrogen inlet. A small amount of naphthalene (0.12 gram) was added followed by the addition of lithium metal (0.49 gram, 70.6 mmol) in small pieces. The mixture quickly became dark reddishbrown and was heated at 40°C for 6 hours. t-Butyl chloride (2.29 grams, 24.8 mmol) was added dropwise at room temperature, and the mixture was stirred for 0.5 hour. 2,2′-Bis(bromomethyl)-1,1′-biphenyl (4.71 grams, 13.9 mmol) in THF (20 ml) was added dropwise, whereupon the color changed from dark red-brown to medium orange. The mixture was allowed to stir overnight at room temperature and was then heated at reflux for 1 hour. Water (40 ml) was added and most of the THF was removed on the steam bath under a stream of nitrogen. The aqueous solution was extracted three times with diethyl ether. The combined organic solution was washed with water. The solvent was evaporated to leave an orange oily residue which was placed in a Kugelrohr distillation apparatus and heated at 220°C 1,33 mbar/ (1 mm Hg) to remove low boiling components, leaving 9.20 grams of an orange glassy solid. $^1$H NMR (benzene-$d_6$) : δ 3.28 (br s, 8H benzylic) ; 6.33-7.33 (complex, 28H, aromatic). $^{31}$P NMR (benzene-$d_6$) : δ + 10.

Example 8 - Preparation of Compound I, 2-Methyl-1-(2-Methyl-phenyl)Naphthalene

Magnesium turnings (4.00 gr, 0.165 mol) and anhydrous diethyl ether (30 mL) were placed in a 500-mL three-neck round bottom flask, fitted with a condenser, addition funnel and nitrogen inlet. A crystal of iodine and 3-4 drops of 1,2-dibromoethane were added and the mixture was stirred magnetically. A solution of 1-bromo-2-methylnaphthalene (33.20 gr, 0.150 mol) in a 1 : 1 mixture of diethyl ether-benzene (120 mL) was

added dropwise at a rate such that gentle reflux was maintained. After the addition was complete (about 1.5 hour), the mixture was heated at reflux for an additional hour, then cooled to room temperature. This mixture was then added rapidly under nitrogen to a stirring mixture of 2-bromotoluene (22.90 gr, 0.134 mol) and bis(triphenylphosphine)nickel dichloride (1.0 gr, 1.52 mmol) in diethyl ether (100 mL). The flask which had contained the Grignard reagent was rinsed with additional diethyl ether (60 mL), which was then added to the reaction mixture. The mixture was heated at reflux for 16 hours and then cooled to room temperature. Water (100 mL) was added, followed by the addition of 20 percent hydrochloric acid (100 mL). After stirring about 1 hour, the mixture was transferred to a separatory funnel and the layers were separated. The organic solution was washed twice with water (100 mL each) and then dried ($MgSO_4$). After filtration, the solvent was evaporated from the filtrate and the residue was distilled to give the desired product (I), boiling point 156°-163° 0,67 mbar/ (0.5 mm Hg), 23.82 gr (77 percent), as a thick, light yellow liquid.

$^1H$ nmr (benzene-$d_6$) : δ = 1.77 (s, $CH_3$), 1.99 (s, CH3), 6.67-7.60 (complex, aromatic)

Example 9 - Preparation of Compound II, 2-Bromomethyl-1-[2-(bromomethyl)phenyl]-naphthalene

2-Methyl-1-(2-methylphenyl)naphthalene (I) (21.58 gr, 0.093 mol) was dissolved in carbon tetrachloride (70 mL) and placed in a 250-mL round bottom flask fitted with a condenser. N-Bromosuccinimide (34.23 gr, 0.192 mol) and N,N'-azobis(isobutyronitrile) (0.1 gr) were added and the mixture was heated at reflux for 5 hr. After cooling to room temperature, the mixture was filtered. The filtrate was washed with saturated aqueous sodium bicarbonate and then was washed twice with saturated aqueous sodium chloride. The organic solution was dried ($MgSO_4$), filtered and evaporated on the rotary evaporator to give a dark amber, thick liquid. This material was dissolved in toluene and passed through a short column of neutral alumina. The solvent was removed on the rotary evaporator to give a thick, orange liquid, 32.34 gr (89 percent), which was used in the next reaction without further purification.

$^1H$ nmr ($CDCl_3$) : δ = 4.03 (dd, $CH_2$), 6.75-7.80 (complex, aromatic)

Example 10 - Preparation of Compound III,
2-(Diphenylphosphinomethyl)-1-[2-(diphenylphosphinomethyl)phenyl]naphthalene Dioxide

The dibromide II (15.10 gr, 0.039 mol) was dissolved in toluene (20 mL) in a 100-mL round bottom flask. A Claisen head, fitted with a short-path distillation head and an addition funnel was attached. The solution of dibromide was heated until toluene began to distill slowly and a solution of methyl diphenylphosphinite (17.56 gr, 0.081 mol) in toluene (23 mL) was added dropwise to the distilling mixture at a rate such that the liquid level in the flask remained approximately constant. After addition was complete, the mixture was heated at reflux an additional 15 minutes. After standing overnight at room temperature, a light brown solid had separated from the solution. The mixture was cooled in an ice bath and then filtered. The solid was washed with cold toluene, followed by diethyl ether to give the dioxide (III) as an off-white powder, 11.12 gr (45 percent).

$^1H$ nmr ($CDCl_3$) : δ = 3.17 (br d, $CH_2$), 6.58-7.83 complex, aromatic)

$^{31}P$ nmr ($CDCl_3$) : δ = −30, −31 ppm

Example 11 - Preparation of PHENAP,
2-(Diphenylphosphinomethyl)-1-[2-(diphenylphosphinomethyl)phenyl] naphthalene

To a cold (ice bath) mixture of the bisphosphine dioxide (III) (4.00 gr, 6.33 mmol) and triethylamine (3.08 gr, 30.51 mmol) in toluene (50 mL), under nitrogen, was added trichlorosilane (4.14 gr, 30.51 mmol), dropwise by syringe. The mixture was allowed to stir overnight at room temperature, then heated at reflux for 3 hours. The resulting heterogeneous mixture was cooled in an ice bath and 20 percent aqueous potassium hydroxide (60 mL) was added gradually. After stirring about 30 minutes at room temperature, the mixture was transferred to a separatory funnel and the layers were separated. The aqueous layer was washed with toluene (40 mL). The organic layers were combined and washed three times with water (40 mL each). The toluene solution was dried ($MgSO_4$) and filtered. The solvent was then removed under vacuum leaving PHENAP as a glassy, yellow solid, 3.64 gr (96 percent).

$^1H$ nmr (benzene-$d_6$) : δ = 3.42 (br d, $CH_2$), 6.58-7.63 (complex, aromatic)

$^{31}P$ nmr (benzene-$d_6$) : δ = + 9, + 12 ppm

Example 12 - Preparation of Rhodium 2-Ethylhexanoate Solution in Texanol® Solvent

The apparatus consists of a 5-liter three-necked flask equipped with a heating mantle, Teflon bladed mechanical stirrer, reflux condenser, and a thermometer. Sodium hydroxide (80 grams) was dissolved in 1,000 ml of water in the flask. 2-Ethylhexanoic acid (196 grams) was added to the flask and dissolved. Rhodium chloride hydrate (46.62 grams containing 20 grams of rhodium metal value) was dissolved in 900 ml of water separately and then added to the stirred sodium 2-ethylhexanoate solution in the flask. The mixture was heated to 95°C and kept vigorously stirred for 1.5 hours. A dark green oil of crude product separated. The mixture was cooled to room temperature and 400 ml of Texanol (2,2,4-trimethyl-pentane-1,3-diol-monoisobutyrate) solvent was added with stirring. The two phases were separated. The aqueous layer was reextracted with three 400 ml Texanol washes which were combined with the first organic extract. The combined organic phases were washed with 1,000 ml of water. The water wash was combined with the original water wash for rhodium analysis. The combined organic phases were filtered through a 0.5-inch thick bed of celite and made up to 2 liters volume with Texanol that was washed through the celite. The concentration of rhodium in the organic phase was 10,000 ppm and in the combined aqueous phase was 2 ppm.

Example 13 - General Bench-Scale Procedure for Low Pressure Hydroformylation of Propylene Using the Invention Catalysts

The reactor consists of a vertically held stainless steel 4 foot by 1 inch (inside diameter) tube having a stainless steel filter element welded into its side near the bottom. The bottom of the tube has a drain valve and the top has a side port through which the vaporized products and unreacted gases leave the reactor. The top end of the tube is provided with a screwed plug which can be removed for charging the catalyst and which contains a thermowell whereby the temperature of the catalyst solution (reaction medium) in the reactor is measured accurately. Hydrogen and carbon monoxide are fed to the reactor from cylinders via pressure regulators and flow controllers which use differential pressure cells and air actuated flow control valves to maintain accurate flow. A third feed of nitrogen from a cylinder goes to the reactor via a pressure regulator and rotameter with needle valve. The carbon monoxide passes through a heated commercial "deoxo" unit as marketed by Engelhard Industries, Division, Engelhard Minerals and Chemicals Corp., Newark, N.J., to remove oxygen impurities. The nitrogen admixed with hydrogen pass through a similar "deoxo" unit before entering the reactor. Propylene is fed as a liquid to a preheater section or plenum chamber, where it is vaporized, then combined with the other feed gases prior to entering the reactor via the stainless steel filter element. The propylene feed rate is measured using rate-of-level drop in a tank containing liquid propylene using an armored rotameter with a needle valve to control the liquid propylene feed rate.

In operation, the catalyst is contained as a solution in the lower portion of the reactor tube and the reactant gases are sparged up through the solution as bubbles emanating from the filter element. Product butyraldehyde is formed in the catalyst solution where it accumulates and eventually is removed as a vapor by vapor/liquid equilibration with unreacted gases. This type of reactor is known as a vapor take-off or gas sparged reactor. The hot gases are cooled upon leaving the reactor through said side port and the butyraldehyde product, along with some unreacted propylene, collects in a cooled high pressure separator connected by suitable conduit means to said side port. The noncondensed gases are let down to atmospheric pressure via a back pressure regulator which controls the reactor pressure. Additional butyraldehyde is condensed out of the atmospheric pressure gas stream by passing it through a series of three dry ice traps. Once an hour the contents of the high pressure separator and dry ice traps are collected and combined. The weight of butyraldehyde product obtained during the hour and its n/iso ratio are calculated using standard gas/liquid chromatographic techniques in combination with the crude weight of the product collected. In practice, approximately one hour is required for this bench unit to reach steady state production rates where catalyst activity and n/iso product ratio remain substantially constant.

Example 14 - Hydroformylation of Propylene with BISBI - Rhodium Catalyst

A catalyst charge was prepared using 2,2'-bis-(diphenylphosphinomethyl)-1,1'-biphenyl (0.40 gram, 0.729 mmol) and rhodium 2-ethylhexanoate solution in TMPDMI (Texanol®) containing 31.25 mg (0.304 mmol) of rhodium as the metal in a total volume of 200 ml of TMPDMI solvent. This preparation was carried out under nitrogen and the catalyst solution charged to the bench unit reactor under argon as described in Example 13. After sealing, the reactor was pressured to 1793 Kpascals (260 psig) with hydrogen, carbon monoxide, and nitrogen and heated to 125°C by anexternal oil bath with said gases purging through the catalyst solution. The gas feed rates at STP were : $H_2$ = GO = 3.36 liters per minute ; and $N_2$ = 0.96 liter

per minute. Propylene feed was then started at 1.92 liters per minute as gas at STP. The run was carried out for a total of 5 hours. Reactor catalyst volume was kept at a standard operating level of 223 ml by pumping in TMPDMI solvent if level drop occurred as measured by a liquid level differential pressure cell attached to the reactor. The average butyraldehyde production rate for the last 4 hours of operation was 82.3 grams per hour, equivalent to a catalyst activity of 5.80 pounds of butyraldehyde per gram Rh-hour (1b HBu/g Rh-hr) with a very high n/iso ratio of 25.1/1.

Example 15 - Comparison of Propylene Hydroformylation Runs Using Different Organophosphine Ligands in the Presence of Rhodium

Table 1 gives data obtained from several runs in the bench unit hydroformylation reactor wherein different organophosphine ligands were used in the presence of rhodium charged as rhodium 2-ethylhexanoate and operated using the same reactor pressure, catalyst volume, and reactant feed rates as described in Example 16. Table 1 shows the utility of the present ligands, and the exceptional utility of the 2,2′-bis(diphenylphosphinomethyl)-1,1′-biphenyl ligand in producing very high n/iso product ratios in the hydroformylation of propylene.

Example 16 - Effect of Hydroformylation Reaction Temperature on Catalyst Activity and n/iso Product Ratios

Table 2 shows the effect of reaction temperature on the catalyst activity and butyraldehyde n/iso ratio obtained from a 2.4/l ligand/rhodium mole ratio of the 2,2′-bis(diphenylphosphinomethyl)-1,1′-biphenyl ligand to rhodium. The reactant feed rates, catalyst volume, and reactor pressure are the same as in Example 16. The data show that the n/iso product ratio increases with lower reaction temperature while the catalyst activity decreases.

TABLE 1

Comparison of Different Organophosphine/Rh Catalyst Systems

| Run No. | Ligand | Rh Charge, mg | Ligand Charge, mmol | L/Rh Mole Ratio | Reactor Temp, °C | Catalyst Activity, lb HBu/g Rh-hr | HBu N/Iso Ratio |
|---|---|---|---|---|---|---|---|
| 1 | BISBI | 31.25 | 0.729 | 2.4/1 | 125 | 5.80 | 25.1/1 |
| 2 | BPBMB | 31.25 | 1.458 | 4.8/1 | 125 | 5.10 | 12.8/1 |
| 3 | BDBMB | 31.25 | 0.729 | 2.4/1 | 125 | 0.87 | 3.64/1 |
| 4 | BIBMB | 31.25 | 1.239 | 4.1/1 | 125 | 1.31 | 2.00/1 |
| 5 | TR-DMCB | 31.25 | 0.729 | 2.4/1 | 125 | 5.05 | 4.36/1 |
| 6 | TR-DMECB | 31.25 | 0.729 | 2.4/1 | 125 | 4.41 | 4.23/1 |
| 7 | TR-DPNOR | 31.25 | 0.729 | 2.4/1 | 125 | 4.07 | 4.32/1 |
| 8 | CIS-DPNOR | 31.25 | 0.729 | 2.4/1 | 125 | 2.44 | 2.67/1 |
| 9 | 1,8-DINAP | 31.25 | 0.729 | 2.4/1 | 125 | 0.84 | 0.95/1 |
| 10 | FL | 31.25 | 0.729 | 2.4/1 | 125 | 5.00 | 3.56/1 |
| 11 | CIS-1,2DPCH | 31.25 | 0.729 | 2.4/1 | 125 | 1.39 | 2.03/1 |
| 12 | DIOP | 31.25 | 0.729 | 2.4/1 | 125 | 5.16 | 4.02/1 |
| 13 | 1,4-BUT | 31.25 | 0.729 | 2.4/1 | 125 | 1.25 | 2.45/1 |
| 14 | 1,3-PROP | 31.25 | 0.729 | 2.4/1 | 125 | 0.97 | 0.84/1 |
| 15 | 1,5-PENT | 31.25 | 0.729 | 2.4/1 | 125 | 2.32 | 2.28/1 |
| 16 | 1,6-HEX | 31.25 | 0.729 | 2.4/1 | 125 | 4.00 | 1.51/1 |

| Run No. | Ligand | Rh Charge, mg | Ligand Charge, mmol | L/Rh Mole Ratio | Reactor Temp. °C | Catalyst Activity, lb HBu/g Rh-hr | HBu N/Iso Ratio |
|---|---|---|---|---|---|---|---|
| 17 | O-XYL | 31.25 | 0.729 | 2.4/1 | 125 | 3.10 | 2.41/1 |
| 18 | TPP | 15.00 | 18.13 | 124/1 | 125 | 9.41 | 2.43/1 |

Above Ligand Identifications

BISBI = 2,2'-bis(diphenylphosphinomethyl)-1,1'-biphenyl
BDBMB = 2,2'-bis(dibenzylphosphinomethyl)-1,1'-biphenyl
BPBMB = 2,2'-bis(phenylbenzylphosphinomethyl)-1,1'-biphenyl
BIBMB = 2,2'-bis(diisobutylphosphinomethyl)-1,1'-biphenyl
TR-DMCB = trans-1,2-bis(diphenylphosphinomethyl)-3,3-dimethylcyclobutane
TR-DMECB = trans-trans-1,2-bis(diphenylphosphinomethyl)-3-ethoxy-4,4-dimethylcyclobutane
TR-DPNOR = trans-2,3-bis(diphenylphosphinomethyl)[2.2.1]bicycloheptane
1,8-DINAP = 1,8-bis(diphenylphosphinomethyl)naphthalene
CIS-DPNOR = endo, cis-2,3-bis(diphenylphosphinomethyl)[2.2.1]bicycloheptane
FL = 1,1'-bis(diphenylphosphino)ferrocene
CIS-1,2DPCH = cis-1,2-bis(diphenylphosphinomethyl)cyclohexane
DIOP = (-)-2,3-O-isopropylidene-2,3-dihydroxy-1,4-bis(diphenylphosphino)butane
1,4-BUT = 1,4-bis(diphenylphosphino)butane
1,3-PROP = 1,3-bis(diphenylphosphino)propane
1,5-PENT = 1,5-bis(diphenylphosphino)pentane
1,6-HEX = 1,6-bis(diphenylphosphino)hexane
O-XYL = α,α'-bis(diphenylphosphino)orthoxylene
TPP = triphenylphosphine

TABLE 2

Effect of Reaction Temperature of BISBI/Rh Catalyst

| Run No. | Rh Charge, mg | Ligand Charge, mmol | L/Rh Mole Ratio | Reactor Temp, °C | Catalyst Activity, lb HBu/g Rh-hr | HBu N/Iso Ratio |
|---|---|---|---|---|---|---|
| 19 | 31.25 | 0.729 | 2.4/1 | 125 | 5.80 | 25.1/1 |
| 20 | 31.25 | 0.729 | 2.4/1 | 115 | 3.43 | 27.2/1 |
| 21 | 31.25 | 0.729 | 2.4/1 | 105 | 2.21 | 28.7/1 |
| 22 | 31.25 | 0.729 | 2.4/1 | 95 | 0.86 | 32.2/1 |

Example 17 - Hydroformylation of Propylene by Rhodium/-PHENAP Oxo Catalyst at 105°C Using a 3/1 Hydrogen/Carbon Monoxide Mole Ratio and 30 Mole Percent Propylene in the Feed

A catalyst solution was prepared by dissolving rhodium (II) 2-ethylhexanoate (containing 33.45 mg of rhodium, 0.325 mmol) in 180 ml of TMPDMI (Texanol®) solvent under nitrogen. PHENAP (0.49 gr, 0.813 mmol) was dissolved in toluene (10 ml) under nitrogen. The PHENAP solution was added to the rhodium/TMPDMI solution and mixed under nitrogen until homogeneous. The catalyst solution was charged to a bench-scale continuous gas sparged reactor as described in Example 13 under an argon blanket. The reactor was pressured to 1793 Kpascals (260 psig) with hydrogen, carbon monoxide, and nitrogen and heated to 105°C. After reaching 105°C, propylene feed was started and the unit was operated at the feed flows listed below as standard temperature and pressure flow rates.

| | |
|---|---|
| Hydrogen | 4.31 Liters/Min |
| Carbon Monoxide | 1.44 Liters/Min |
| Propylene | 2.88 Liters/Min |
| Nitrogen | 0.96 Liter/Min |

The reactor was run under these conditions for a total of 5 hours with the butyraldehyde product collected, weighed, and analyzed by gas/liquid chromatography hourly. The net weight of butyraldehyde product averaged 100.9 grams per hour in the last 4 hours of operation. The mole ratio of normal butyraldehyde/isobutyraldehyde (n/iso ratio) over this period averaged 54.3/1. The oxo activity of the catalyst is equivalent to 6.65 pounds butyraldehyde per gram Rh-hour.

In the following Example 18, several other ligands were used under essentially the same conditions as this Example 17 to hydroformylate propylene. The runs of Examples 17 and 18 are designated (a)-(o) in Table 3 and are compared therein to show the vast improvement in n/iso ratio achieved by the present invention.

Example 18 - Hydroformylation of Propylene : Comparison of Ligands

For runs (b)-(o), the reactor was maintained at 125°C and 1793 Kpascals (260 psig) with the catalyst charges shown in the table. The reactant gases were fed to the reactor at the rates shown below for 5 hours.

| | |
|---|---|
| Hydrogen | 3.36 Liters/Min |
| Carbon Monoxide | 3.36 Liters/Min |
| Propylene | 1.92 Liters/Min |
| Nitrogen | 0.96 Liter/Min |

Butyraldehyde product was collected, weighed, and analyzed as in Example 17.

TABLE 3

Comparison of Different Ligand/Rh Catalyst Systems at 260 psig

| Run | Ligand | Rh Charge, mg | Ligand Charge, mmol | L/Rh Mole Ratio | Reactor Temp. °C | Catalyst Activity, lb HBu/g Rh-hr | HBu N/Iso Ratio |
|---|---|---|---|---|---|---|---|
| (a) | PHENAP | 33.45 | 0.813 | 2.5/1 | 125 | 6.65 | 54.3/1 |
| (b) | TR-DMCB | 31.25 | 0.729 | 2.4/1 | 125 | 5.05 | 4.36/1 |
| (c) | TR-DMECB | 31.25 | 0.729 | 2.4/1 | 125 | 4.41 | 4.23/1 |
| (d) | TR-DPNOR | 31.25 | 0.729 | 2.4/1 | 125 | 4.07 | 4.32/1 |
| (e) | CIS-DPNOR | 31.25 | 0.729 | 2.4/1 | 125 | 2.44 | 2.67/1 |
| (f) | 1,8-DINAP | 31.25 | 0.729 | 2.4/1 | 125 | 0.84 | 0.95/1 |
| (g) | FL | 31.25 | 0.729 | 2,4/1 | 125 | 5.00 | 3.56/1 |
| (h) | CIS-1,2DPCH | 31.25 | 0.729 | 2.4/1 | 125 | 1.39 | 2.03/1 |
| (i) | DIOP | 31.25 | 0.729 | 2.4/1 | 125 | 5.16 | 4.02/1 |
| (j) | 1,4-BUT | 31.25 | 0.729 | 2.4/1 | 125 | 1.25 | 2.45/1 |
| (k) | 1,3-PROP | 31.25 | 0.729 | 2.4/1 | 125 | 0.97 | 0.84/1 |
| (l) | 1,5-PENT | 31.25 | 0.729 | 2.4/1 | 125 | 2.32 | 2.28/1 |
| (m) | 1,6-HEX | 31.25 | 0.729 | 2.4/1 | 125 | 4.00 | 1.51/1 |

| Run | Ligand | Rh Charge, mg | Ligand Charge, mmol | L/Rh Mole Ratio | Reactor Temp. °C | Catalyst Activity, lb HBu/g Rh-hr | HBu N/Iso Ratio |
|---|---|---|---|---|---|---|---|
| (n) | O-XYL | 31.25 | 0.729 | 2.4/1 | 125 | 3.10 | 2.41/1 |
| (o) | TPP | 15.00 | 18.13 | 124/1 | 125 | 9.41 | 2.43/1 |

Above Ligand Identifications

TR-DMCB = trans-1,2-bis(diphenylphosphinomethyl)-3,3-dimethylcyclobutane
TR-DMECB = trans-trans-1,2-bis(diphenylphosphinomethyl)-3-ethoxy-4,4-dimethylcyclobutane
TR-DPNOR = trans-2,3-bis(diphenylphosphinomethyl)[2.2.1]bicycloheptane
1,8-DINAP = 1,8-bis(diphenylphosphinomethyl)naphthalene
CIS-DPNOR = endo, cis-2,3-bis(diphenylphosphinomethyl)[2.2.1]bicycloheptane
FL = 1,1'-bis(diphenylphosphino)ferrocene
CIS-1,2DPCH = cis-1,2-bis(diphenylphosphinomethyl)cyclohexane
DIOP = (-)-2,3-O-isopropylidene-2,3-dihydroxy-1,4-bis(diphenylphosphino)butane
1,4-BUT = 1,4-bis(diphenylphosphino)butane
1,3-PROP = 1,3-bis(diphenylphosphino)propane
1,5-PENT = 1,5-bis(diphenylphosphino)pentane
1,6-HEX = 1,6-bis(diphenylphosphino)hexane
O-XYL = α,α'-bis(diphenylphosphino)orthoxylene
TPP = triphenylphosphine

Example 19 - Hydroformylation of Propylene by Rhodium/-NAPHOS Oxo Catalyst at 105°C Using a 3/1 Hydrogen/Carbon Monoxide Mole Ratio and 30 Mol Percent Propylene in the Feed

A catalyst solution was prepared by dissolving rhodium (II) 2-ethylhexanoate (containing 33.45 mg of rhodium ; 0.325 mmole) in 180 ml of TMPDMI solvent under nitrogen. NAPHOS (0.53 gram ; 0.813 mmole) was dissolved in 10 ml of toluene under nitrogen. The NAPHOS solution was added to the rhodium/TMPDMI solution and mixed under nitrogen until homogeneous. The catalyst solution was charged to the bench scale continuous gas-stripped reactor under an argon blanket. The reactor was pressured to 1793 Kpascals (260 psig) with hydrogen, carbon monoxide, and nitrogen and heated to 105°C. After reaching 105°C, propylene feed was started and the unit was operated at the feed rates listed below at standard temperature and pressure conditions, in Liters/Minute.

| | |
|---|---|
| Hydrogen | 4.31 Liters/Min |
| Carbon Monoxide | 1.44 Liters/Min |
| Propylene | 2.88 Liters/Min |
| Nitrogen | 0.96 Liter/Min |

The reactor was run under these conditions for a total of 5 hours with the butyraldehyde product collected, weighed, and analyzed by gas/liquid chromatography hourly. The net weight of butyraldehyde product averaged 124.6 grams-per hour in the last 4 hours of operation. The mole ratio of normal butyraldehyde/isobutyraldehyde (n/iso ratio) over this period averaged 56.6/l. The oxo activity of the catalyst is equivalent to 8.2 pounds butyraldehyde per gram Rh-hour.

In the following Example 20, NAPHOS and several other ligands were used under essentially identical conditions to hydroformylate propylene. The runs are designated (a)-(o) in Table 4 and are given to show the vast improvement in n/Iso ratio achieved by the present invention.

Example 20

The catalyst charge from Example 19 employing NAPHOS ligand was left in the reactor under 1793 Kpascals (260 psig) of hydrogen and carbon monoxide pressure overnight at room temperature. The reactor was heated to 125°C and run at 1793 Kpascals (260 psig). The reactant gases were fed to the reactor at the rates shown below for 5 hours.

| | |
|---|---|
| Hydrogen | 3.36 Liters/Min |
| Carbon Monoxide | 3.36 Liters/Min |
| Propylene | 1.92 Liters/Min |
| Nitrogen | 0.96 Liter/Min |

Butyraldehyde product was collected, weighed, and analyzed as in Example 19. The net weight of butyraldehyde product averaged 88.9 grams per hour in the last 4 hours of operation with a n/iso ratio of 20.4/l. The oxo activity of the catalyst is equivalent to 5.85 pounds of butyraldehyde per gram Rh-hour. This run is designated (a) in Table 4. Runs (b)-(o) employing different ligands were made at essentially these same reactant feed rates and at the catalyst charges shown in the Table.

Example 21 - Hydroformylation of 1-Octene by Rhodium/NAPHOS

A solution of rhodium (I) dicarbonylacetylacetonate (0.0377 gram ; 0.146 mmole), NAPHOS (0.23 gram; 0.35 mmole) and octene-1 (31.3 ml, 22.4 grams ; 0.2 mole) in toluene (69 ml) was prepared under nitrogen and placed in a nitrogen flushed 300-ml stainless steel autoclave. The autoclave was pressurized to 2070 Kpascals (300 psig) with 1/1 $H_2/CO$ and heated, with rapid stirring, to 105°C. When the pressure dropped to 1724 Kpascals (250 psig), it was replenished to 2070 Kpascals (300 psig) with 1/1, $H_2/CO$. The reaction was maintained at 105°C and 1724-2070 Kpascals (250-300 psig) for 2 hours. A total pressure drop of 325 pounds was observed. After cooling to room temperature, the light yellow solution was removed from the autoclave and analyzed by gas chromatography. Results are shown in Table 5 in runs (a)-(j) wherein a variety of other ligands were also used under the same reaction conditions except where the ligand/Rh mole ratio is shown to be different. The very large improvement in n/Iso ratio for the product aldehydes as a result of the use of NAPHOS ligand is shown in Table 6, run (j).

Example 22 - Hydroformylation of Allyl Alcohol by Rhodium/NAPHOS

A solution of rhodium (I) dicarbonylacetylacetonate (0.0377 gram ; 0.146 mmole), NAPHOS (0.23 gram; 0.35 mmole) and allyl alcohol (13.6 ml ; 11.6 grams ; 0.2 mole) in toluene (86 ml) was prepared under nitrogen and placed in a nitrogen flushed 300-ml stainless steel autoclave. The autoclave was pressurized to 862 Kpascals (125 psig) with $H_2/CO$ (1/1 molar ratio) and heated, with rapid stirring, to 80°C. When the pressure dropped to 517 Kpascals (75 psig), it was replenished to 862 Kpascals (125 psig) with additional (1/1) $H_2/CO$. The reaction was maintained at 80°C and 517-862 Kpascals (75-125 psig) for 30 minutes. A total pressure drop of 305 pounds was observed. After cooling to room temperature, the light yellow solution was removed from the autoclave and analyzed by gas chromatography. Conversion of allyl alcohol : 98.7 percent. Yields (based on allyl) alcohol) : 4-hydroxybutyraldehyde –81.3 percent, 2-methyl-3-hydroxypropionaldehyde –13.0 percent, propionaldehyde-4.4 percent. Ratio of linear oxo aldehyde to branched oxo aldehyde - 6.28/l.

TABLE 4

Comparison of Different Ligand/Rh Catalyst Systems at 260 psig

| Run | Ligand | Rh Charge, mg | Ligand Charge, mmol | L/Rh Mole Ratio | Reactor Temp, °C | Catalyst Activity, lb HBu/g Rh-hr | HBu N/Iso Ratio |
|---|---|---|---|---|---|---|---|
| (a) | NAPHOS | 33.45 | 0.813 | 2.5/1 | 125 | 5.85 | 20.4/1 |
| (b) | TR-DMCB | 31.25 | 0.729 | 2.4/1 | 125 | 5.05 | 4.36/1 |
| (c) | TR-DMECB | 31.25 | 0.729 | 2.4/1 | 125 | 4.41 | 4.23/1 |
| (d) | TR-DPNOR | 31.25 | 0.729 | 2.4/1 | 125 | 4.07 | 4.32/1 |
| (e) | CIS-DPNOR | 31.25 | 0.729 | 2.4/1 | 125 | 2.44 | 2.67/1 |
| (f) | 1,8-DINAP | 31.25 | 0.729 | 2.4/1 | 125 | 0.84 | 0.95/1 |
| (g) | FL | 31.25 | 0.729 | 2.4/1 | 125 | 5.00 | 3.56/1 |
| (h) | CIS-1,2DPCH | 31.25 | 0.729 | 2.4/1 | 125 | 1.39 | 2.03/1 |
| (i) | DIOP | 31.25 | 0.729 | 2.4/1 | 125 | 5.16 | 4.02/1 |
| (j) | 1,4-BUT | 31.25 | 0.729 | 2.4/1 | 125 | 1.25 | 2.45/1 |
| (k) | 1,3-PROP | 31.25 | 0.729 | 2.4/1 | 125 | 0.97 | 0.84/1 |
| (l) | 1,5-PENT | 31.25 | 0.729 | 2.4/1 | 125 | 2.32 | 2.28/1 |
| (m) | 1,6-HEX | 31.25 | 0.729 | 2.4/1 | 125 | 4.00 | 1.51/1 |

| Run | Ligand | Rh Charge, mg | Ligand Charge, mmol | L/Rh Mole Ratio | Reactor Temp, °C | Catalyst Activity, lb HBu/g Rh-hr | HBu N/Iso Ratio |
|---|---|---|---|---|---|---|---|
| (n) | O-XYL | 31.25 | 0.729 | 2.4/1 | 125 | 3.10 | 2.41/1 |
| (o) | TPP | 15.00 | 18.13 | 124/1 | 125 | 9.41 | 2.43/1 |

Above Ligand Identifications

TR-DMCB = trans-1,2-bis(diphenylphosphinomethyl)-3,3-dimethylcyclobutane
TR-DMECB = trans-trans-1,2-bis(diphenylphosphinomethyl)-3-ethoxy-4,4-dimethylcyclobutane
TR-DPNOR = trans-2,3-bis(diphenylphosphinomethyl)[2.2.1]bicycloheptane
1,8-DINAP = 1,8-bis(diphenylphosphinomethyl)naphthalene
CIS-DPNOR = endo, cis-2,3-bis(diphenylphosphinomethyl)[2.2.1]bicycloheptane
FL = 1,1'-bis(diphenylphosphino)ferrocene
CIS-1,2DPCH = cis-1,2-bis(diphenylphosphinomethyl)cyclohexane
DIOP = (-)-2,3-O-isopropylidene-2,3-dihydroxy-1,4-bis(diphenylphosphino)butane
1,4-BUT = 1,4-bis(diphenylphosphino)butane
1,3-PROP = 1,3-bis(diphenylphosphino)propane
1,5-PENT = 1,5-bis(diphenylphosphino)pentane
1,6-HEX = 1,6-bis(diphenylphosphino)hexane
O-XYL = α,α'-bis(diphenylphosphino)orthoxylene
TPP = triphenylphosphine

TABLE 5

Batch Autoclave Hydroformylations of Octene-1

| Run | Ligand | L/Rh Ratio | % Conversion Octene-1 | % Yield to Isooctene[b] | % Yield to $C^9$ Aldehyde of Olefin Fed |
|---|---|---|---|---|---|
| (a) | O-XYL | 2.4/1 | 94.3 | 3.7 | 90.6 |
| (b) | TR-DMCB | 2.4/1 | 97.1 | 2.8 | 94.3 |
| (c) | FL | 2.4/1 | 93.6 | 3.1 | 90.5 |
| (d) | TPP | 150/1 | 98.4 | 3.5 | 94.9 |
| (e) | DIOP | 2.4/1 | 98.1 | 4.3 | 93.8 |
| (F) | BDPP[a] | 5/1 | 99.2 | 2.9 | 96.3 |
| (g) | 1,4-BUT | 2.4/1 | 95.3 | 2.7 | 92.6 |
| (h) | 1,5-PENT | 2.4/1 | 98.4 | 2.6 | 95.8 |
| (i) | 1,3-PROP | 2.4/1 | 67.0 | 1.9 | 65.1 |
| (j) | NAPHOS | 2.4/1 | 98.1 | 7.2 | 90.9 |

[a]BDPP – n-Butyldiphenylphosphine.
[b]Mixture of octene-2 and octene-3 isomers.

TABLE 6

% Isomer Distribution of $C_9$ Aldehyde

| Run | n-Nonanal | 2-Methyl octanal | 2-Ethyl heptanal | 2-Propyl hexanal | N/Branched Aldehyde Ratio |
|---|---|---|---|---|---|
| (a) | 77.64 | 22.32 | 0.05 | 0.00 | 3.47/1 |
| (b) | 87.2 | 12.74 | 0.06 | 0.00 | 6.81/1 |
| (c) | 82.8 | 17.19 | 0.05 | 0.00 | 4.80/1 |
| (d) | 77.2 | 21.49 | 0.77 | 0.04 | 3.46/1 |
| (e) | 85.12 | 14.70 | 0.189 | 0.00 | 5.72/1 |
| (f) | 72.82 | 25.02 | 1.99 | 0.164 | 2.68/1 |
| (g) | 75.16 | 24.75 | 0.081 | 0.00 | 3.03/1 |
| (h) | 73.35 | 26.14 | 0.507 | 0.00 | 2.75/1 |
| (i) | 49.10 | 50.78 | 0.115 | 0.00 | 0.96/1 |
| (j) | 97.11 | 2.73 | 0.16 | 0.00 | 33.60/1 |

The invention has been described in detail with particular reference to preferred embodiments thereof, but it will be understood that variations and modifications can be effected within the spirit and scope of the invention.

## Claims

1. A hydroformylation process comprising contacting hydroformylation stock in a reaction zone at a temperature of from 20°C to 250°C and a pressure of from 103 Kpascals to 5517 Kpascals (15 psig to 800 psig) with hydrogen, carbon monoxide, and a catalyst comprising rhodium in chemical complex with one or more ligands of the formula

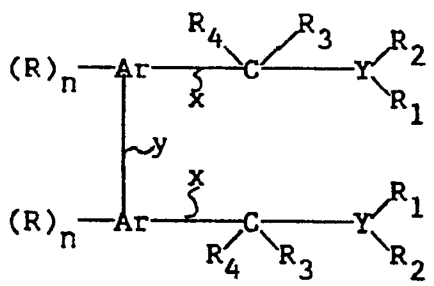

wherein :

each Ar is independently selected from aromatic ring compounds having 6 up to 14 carbon atoms ;

the x bonds and the y bonds are attached to adjacent carbon atoms on the ring structures ;

each R, when present as a substituent, is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

n is a whole number in the range of 0-4 were Ar is phenyl ; 0-6 where Ar is naphthyl ; and 0-8 where Ar is phenanthryl or anthracenyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each of the above alkyl groups or moieties is straight or branched chain of 1-20 carbons, preferably 1-8 carbons ;

each aryl group contains 6-10 ring carbons ;

each cycloaliphatic group contains from 4-8 ring carbons ; and

each Y is independently selected from the elements N, P, As, Sb and Bi,

for a sufficient period of time to permit reaction of said olefin with said carbon monoxide and hydrogen to form aldehyde product.

2. The hydroformylation process according to Claim 1 wherein said ligand has the formula :

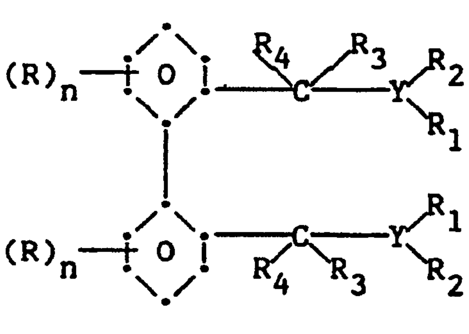

wherein :

n is 0-4 ;

each R is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

wherein each alkyl group or moiety is straight or branched chain of 1-20 carbons, each aryl group contains 6-10 ring carbons, and each cycloaliphatic group contains from 4-8 ring carbons ; and

each Y is independently selected from the elements N, P, As, Sb and Bi.

3. The hydroformylation process according to Claim 1 wherein said ligand has the formula :

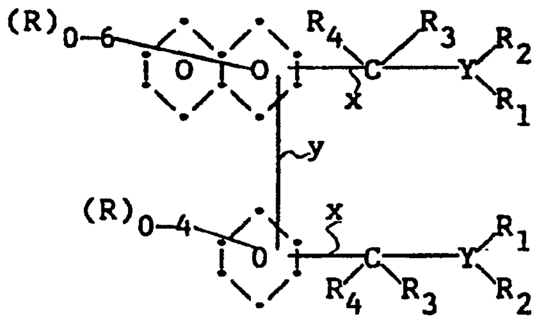

wherein :

the x bonds and the y bonds are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

wherein each of the above alkyl groups or moieties is straight or branched chain of 1-20 carbons, each aryl group contains 6-10 ring carbons, and each cycloaliphatic group contains from 4-8 ring carbons ; and

each Y is independently selected from the elements N, P, As, Sb and Bi.

4. The hydroformylation process according to Claim 1 wherein said ligand has the formula :

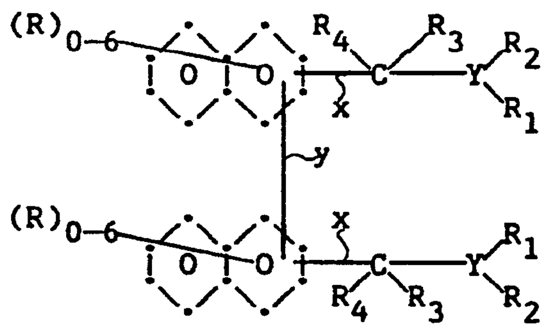

wherein :

the x bonds and the y bonds are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

wherein each of the above alkyl groups or moieties is straight or branched chain of 1-20 carbons, each aryl group contains 6-10 ring carbons, and each cycloaliphatic group contains from 4-8 ring carbons ; and

each Y is independently selected from the elements N, P, As, Sb and Bi.

5. The process of Claim 1 wherein each said alkyl group or moiety is from 1-8 carbons.

6. The hydroformylation process according to Claim 1 wherein said reaction zone is operated at temperatures between about 80°C and 150°C and at pressures between about 100 psig and 400 psig, and the

molar ratio of ligand to rhodium is from about 0.5 to about 200.

7. The hydroformylation process according to Claim 6 wherein the molar ratio of said hydrogen to carbon monoxide is at least 0.5, and the total moles of hydrogen and carbon monoxide are present in said reaction zone in the ratio range of from about 0.02 to about 20 with respect to moles of said olefin.

8. The hydroformylation process according to Claim 7 wherein said hydroformylation stock is selected from one or more of ethylene, propylene, 2-methylpropylene, 2-butene, 1-butene, 2-methyl-1-butene, 1-pentene, 1-hexene, 1-heptene, 1-octene, allyl alcohol, allyl acetate, methyl vinyl ether, ethyl vinyl ether, allyl ethyl ether, vinyl acetate and acrolein diethyl acetal.

9. The hydroformylation process according to Claim 1 wherein said rhodium is present in said reaction zone in an amount between $1 \times 10^{-6}$ to $1 \times 10^{-1}$ moles per mole of said olefin present in said reaction zone.

10. The process of Claim 2 wherein said ligand is selected from : 2,2'-bis(diphenylphosphinomethyl)-1,1'-biphenyl ; 2,2'-bis-(dibenzylphosphinomethyl)-1,1'-biphenyl ; 2,2'-bis(phenylbenzylphosphinomethyl)-1,1'-biphenyl ; 2,2'-bis-(diisobutylphosphinomethyl)-1,1'-biphenyl ; or mixtures thereof.

11. The process of Claim 10 wherein the olefin is propylene.

12. The process of Claim 10 wherein the molar ratio of ligand to rhodium is from 2 to 10.

13. The process of Claim 10 wherein the molar ratio of ligand to rhodium is from 2.3 to 4.

14. The process of Claim 1 wherein at steady state hydroformylation conditions in said reaction zone the molar ratio of ligand to Rh is from 2 to 5, the ratio of Rh(mg.)/solvent (ml) is from 0.07 to 0.28, the ratio of [olefin feed in L(STP)/min]/mg. of Rh is from 0.03 to 0.30, the ratio of [CO or $H_2$ feed in L(STP)/min]/mg. of Rh is from 0.015 to 1.5, the temperature is maintained at from 80°C to 150°C and the reactor pressure is maintained at from 1655 Kpascals to 1931 Kpascals (240 psig to 280 psig).

15. The process of any one of Claims 2-9 or 14 wherein the ligand is 2,2'-bis(diphenylphosphinomethyl)-1,1'-biphenyl.

16. The process of Claim 3 wherein said ligand has the formula

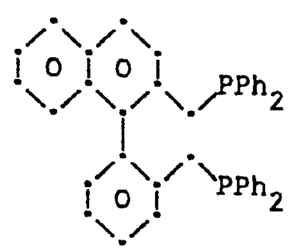

17. The process of Claim 16 wherein the olefin is propylene.

18. The process of Claim 16 wherein the molar ratio of ligand to rhodium is from 1 to 10.

19. The process of Claim 16 wherein the molar ratio of ligand to rhodium is from 2.3 to 4.

20. The process of Claim 4 wherein said ligand has the formula

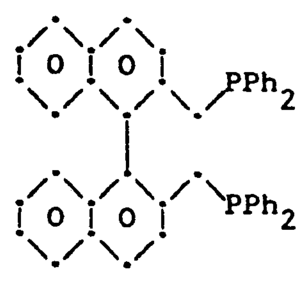

21. The process of Claim 20 wherein the olefin is propylene.

22. The process of Claim 20 wherein the molar ratio of ligand to rhodium is from 1 to 10.

23. The process of Claim 20 wherein the molar ratio of ligand to rhodium is from 2.3 to 4.

24. A compound of the formula

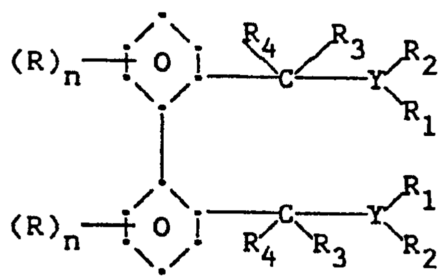

useful as a ligand in hydroformylation and other reactions, wherein

n is 0-4 ;

each R is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each Y is independently selected from the elements N, P, As, Sb and Bi ; and

each alkyl group or moiety is straight or branched chain of 1-20 carbons, each aryl group contains 6-10 ring carbons, and each cycloaliphatic group contains from 4-8 ring carbons.

25. The compound of Claim 24 wherein n is zero, $R_1$ and $R_2$ are alkyl or phenyl, and $R_3$ and $R_4$ are hydrogen or phenyl.

26. The compound of Claim 24 selected from : 2,2′-bis(diphenylphosphinomethyl)-1,1′-biphenyl ; 2,2′-bis(dibenzylphosphinomethyl)-1,1′-biphenyl ; 2,2′-bis(phenylbenzylphosphinomethyl)-1,1′-biphenyl ; 2,2′-bis(diisobutylphosphinomethyl)-1,1′-biphenyl ; and mixtures thereof.

27. The compound of Claim 24 designated 2,2′-bis-(diphenylphosphinomethyl)-1,1′-biphenyl.

28. A compound of the formula

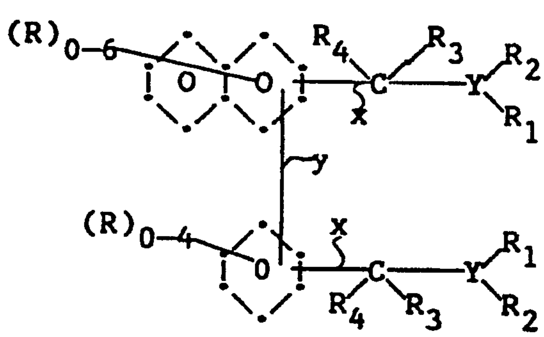

wherein :

the x bonds and the y bond are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each Y is independently selected from the elements N, P, As, Sb and Bi ; and

each of the alkyl groups or moieties is straight or branched chain of 1-20 carbons, each aryl group contains 6-10 ring carbons, and each cycloaliphatic group contains from 4-8 ring carbons.

29. A compound of Claim 28 wherein each said alkyl group or moiety is from 1-8 carbons.

30. A compound of Claim 28 wherein the rings are unsubstituted, $R_1$ and $R_2$ are alkyl or phenyl, and $R_3$ and $R_4$ are hydrogen or phenyl.

31. A compound of Claim 28 of the structure

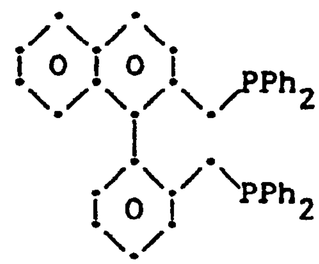

32. The catalyst comprising rhodium complexed with : (a) a ligand of the formula

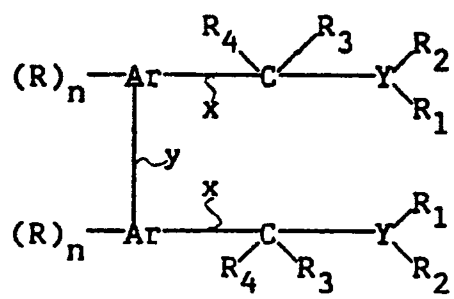

wherein :

each Ar is independently selected from aromatic ring compounds having 6 up to 14 carbon atoms, e.g., phenyl, naphthyl, phenanthryl and anthracenyl ;

the x bonds and the y bonds are attached to adjacent carbon atoms on the ring structures ;

each R, when present as a substituent, is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

n is a whole number in the range of 0-4 were Ar is phenyl ; 0-6 where Ar is naphthyl ; and 0-8 where Ar is phenanthryl or anthracenyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each of the above alkyl groups or moieties is straight or branched chain of 1-20 carbons, preferably 1-8 carbons ;

each aryl group contains 6-10 ring carbons ;

each cycloaliphatic group contains from 4-8 ring carbons ; and

each Y is independently selected from the elements N, P, As, Sb and Bi ;

in a molar ratio of ligand/Rh of 1/1 ;

(b) H in an atomic ratio of H/Rh of 1/1 ; and

(c) carbon monoxide in a molar ratio of CO/Rh of 2/1.

33. The catalyst according to Claim 32 wherein said ligand has the formula :

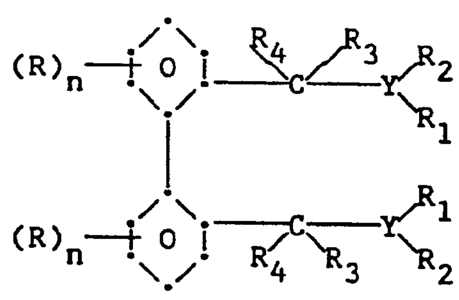

wherein

n is 0-4 ;

each R is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

each Y is independently selected from the elements N, P, As, Sb and Bi ; and

each alkyl group or moiety is straight or branched chain of 1-20 carbons, each aryl group contains 6-10 ring carbons, and each cycloaliphatic group contains from 4-8 ring carbons.

34. The catalyst according to Claim 33 wherein the ligand is selected from : 2,2'-bis(diphenylphosphinomethyl)-1,1'-biphenyl ; 2,2'-bis-(dibenzylphosphinomethyl)-1,1'-biphenyl ; 2,2'-bis(phenylbenzylphosphinomethyl)-1,1'-biphenyl ; 2,2'-bis(diisobutylphosphinomethyl)-1,1'-biphenyl ; and mixtures thereof.

35. The catalyst according to Claim 32 wherein said ligand has the formula :

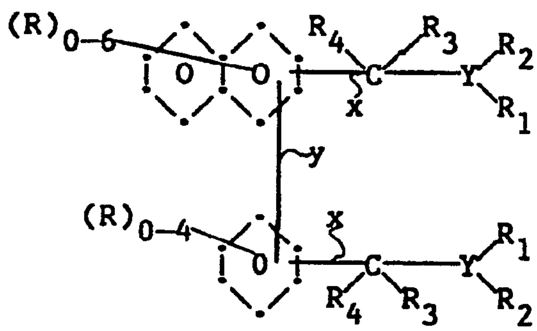

wherein :

the x bonds and the y bonds are attached to adjacent carbon atoms on the ring structures ;

each R when present as a substituent is independently selected from alkyl, alkoxy, aryloxy, aryl, aralkyl, alkaryl, alkoxyalkyl, cycloaliphatic, halogen (except C1, Br or I in the ortho position), alkanoyl, alkanoyloxy, alkoxycarbonyl, or formyl ;

each $R_1$ and $R_2$ is independently selected from alkyl, aryl, aralkyl, alkaryl or cycloaliphatic ;

each $R_3$ and $R_4$ is independently selected from hydrogen and the $R_1$ substituents ;

wherein each of the above alkyl groups or moieties is straight or branched chain of 1-20 carbons, each aryl group contains 6-10 ring carbons, and each cycloaliphatic group contains from 4-8 ring carbons ; and

each Y is independently selected from the elements N, P, As, Sb and Bi.

36. The catalyst according to Claim 35 wherein the ligand has the structure

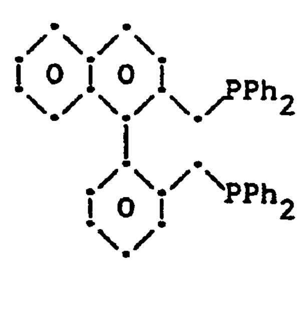

**Ansprüche**

1. Hydroformylierungsverfahren, bei dem man das zu hydroformylierende Material in einer Reaktionszone bei einer Temperatur von 20°C bis 250°C und einem Druck von 103 KPascal bis 5517 KPascal mit Wasserstoff, Kohlenmonoxid und einem Katalysator mit Rhodium in chemischem Komplex mit einem Oder mehreren Liganden der Formel

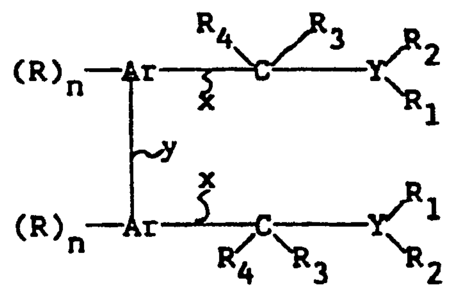

in der bedeuten :

Ar jeweils unabhängig voneinander eine aromatische Ringverbindung mit 6 bis 14 Kohlenstoffatomen;

die x-Bindungen und y-Bindungen sind an benachbarte Kohlenstoffatome der Ringstrukturen gebunden ;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in der ortho-Position), Alkanoyl, Alkanoyloxy, Alkoxycarbonyl oder Formyl ;

n eine ganze Zahl von 0-4, wenn Ar für Phenyl steht ; 0-6 ist, wenn Ar für Naphthyl steht ; und 0-8 ist, wenn Ar für Phenanthryl oder Anthracenyl steht ;

$R_1$ und $R_2$ jeweils unabhängig voneinander Alkyl, Aryl, Aralkyl, Alkaryl oder eine cycloaliphatische Gruppe ;

$R_3$ und $R_4$, jeweils unabhängig voneinander Wasserstoff oder ein Substituent $R_1$ ;

wobei jede der Alkylgruppen oder jeder der Reste eine gerade oder verzweigte Rette mit 1-20 Kohlenstoffatomen, vorzugsweise 1-8 Kohlenstoffatomen ist,

jede Arylgruppe 6-10 Ringkohlenstoffatome aufweist ;

jede cycloaliphatische Gruppe 4-8 Ringkohlenstoffatome aufweist und

jedes Y unabhängig voneinander ausgewählt ist aus den Elementen N, P, As, Sb und Bi,

eine solche Zeitspanne lang in Kontakt miteinander bringt, daß eine Umsetzung des Olefins mit dem Kohlenmonoxid und Wasserstoff unter Bildung eines Aldehydproduktes stattfindet.

2. Hydroformylierungsverfahren nach Anspruch 1, bei dem der Ligand der folgenden Formel entspricht:

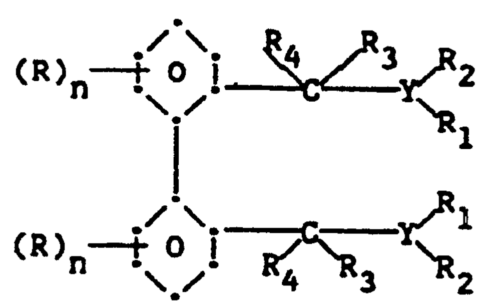

in der bedeuten :

n eine Zahl von 0-4 ;

R jeweils unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br, oder I in ortho-Position), Alkanoyl, Alkanoyloxy, Alkoxycarbonyl, oder Formyl ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneinander Wasserstoff oder einen R, Substituenten ;

wobei jede Alkylgruppe oder jeder Rest eine gerade oder verzweigte Kette mit 1-20 Kohlenstoffatomen

ist ; jede Arylgruppe 6-10 Kohlenstoffatome aufweist und jede cycloaliphatische Gruppe 4-8 Ringkohlenstofftome enthält ; und

Y jeweils unabhängig voneinander ausgewählt ist aus den Elementen N, P, As, Sb und Bi.

3. Hydroformulyierungsverfahren nach Anspruch 1, bei dem der Ligand der folgenden Formel entspricht :

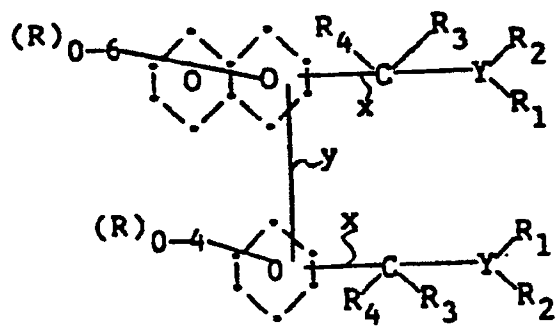

in der

die x-Bindungen und y-Bindungen an benachbarte Kohlenstoffatome der Ringstrukturen gebunden sind ;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br, oder I in der ortho-Position), Alkanoyl, Alkanoyloxy, Alkoxycarbonyl oder Formyl ;

R, und $R_2$ jeweils unabhängig voneinander Alkyl, Aryl, Aralkyl, Alkaryl oder eine cycloaliphatische Gruppe ;

$R_3$ und $R_4$, jeweils unabhängig voneinander Wasserstoff oder ein Substituent $R_1$ ;

wobei jede der Alkylgruppen oder jeder der Reste eine gerade oder verzweigte Kette mit 1-20 Kohlenstoffatomen, vorzugsweise 1-8 Kohlenstoffatomen ist, jede Arylgruppe 6-10 Ringkohlenstoffatome und jede cycloaliphatische Gruppe 4-8 Ringkohlenstoffatome aufweist und

jedes Y unabhängig voneinander ausgewählt ist aus den Elementen N, P, As, Sb und Bi.

4. Hydroformylierungsverfahren nach Anspruch 1, bei dem der Ligand der folgenden Formel entspricht:

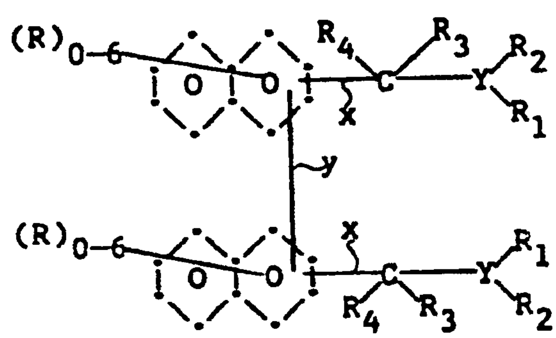

in der bedeuten :

die x-Bindungen und y-Bindungen sind an benachbarte Kohlenstoffatome der Ringstruktur gebunden;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in der ortho-Position), Alkanoyl, Alkanoyloxy, Alkoxycarbonyl oder Formyl ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneinander Wasserstoff oder ein Substituent $R_1$ ;

wobei jede der Alkylgruppen oder jeder der Reste eine gerade oder verzweigte Kette mit 1-20 Kohlenstoffatomen, vorzugsweise 1-8 Kohlenstoffatomen ist, jede Arylgruppe 6-10 Ringkohlenstoffatome aufweist ; jede cycloaliphatische Gruppe 4-8 Ringkohlenstoffatome aufweist und

jedes Y unabhängig voneinander ausgewählt ist aus den Elementen N, P, As, Sb und Bi.

5. Verfahren nach Anspruch 1, in dem jede Alkylgruppe oder jede Gruppe 1-8 Kohlenstoffatome aufweist.

6. Hydroformylierungsverfahren nach Anspruch 1, in dem in der Reaktionszone Temperaturen von etwa 80°C bis 150°C und Drücke zwischen etwa 690 bis etwa 2760 KPascal herrschen und das Molverhältnis von Liganden zu Podium bei etwa 0,5 bis etwa 200 liegt.

7. Hydrofomulyierungsverfahren nach Anspruch 6, in dem das Molverhältnis von Wasserstoff zu Kohlenmonoxid mindestens 0,5 beträgt und die Gesamtmole an Wasserstoff und Kohlenmonoxid in der Reaktionszone im Verhältnis von etwa 0,02 bis etwa 20 bezüglich Molen an Olefin vorliegen.

8. Hydroformylierungsverfahren nach Anspruch 7, in dem das zu hydroformylierende Material ausgewählt ist aus einer oder mehreren der folgenden Verbindungen : Ethylen, Propylen, 2-Methylpropylen, 2-Buten, 1-Buten, 2-Methyl-1-buten, 1-Penten, 1-Hexen, 1-Hepten, 1-Octen, Allylalkohol, Allylacetat, Methylvinylether, Ethylvinylether, Allylethylether, Vinylacetat und Acroleindiethylacetal.

9. Hydroformylierungsverfahren nach Anspruch 1, in dem Rhodium in der Reaktionszone in einer Konzentration von $1 \times 10^{-6}$ bis $1 \times 10^{-1}$ Molen pro Mol in der Reaktionszone vorliegendes Mol Olefin vorliegt.

10. Verfahren nach Anspruch 2, in dem der Ligand ausgewählt ist aus : 2,2'-Bis(diphenylphosphinomethyl)-1,1'-biphenyl ; 2,2'-Bis-(dibenzylphosphinomethyl)-1,1'-biphenyl ; 2,2'-Bis(phenylbenzylphosphinomethyl)-1,1'-biphenyl ; 2,2'-Bis-(diisobutylphosphinomethyl)-1,1'-biphenyl ; oder Mischungen hiervon.

11. Verfahren nach Anspruch 10, in dem als Olefin Propylen eingesetzt wird.

12. Verfahren nach Anspruch 10, in dem das Molverhältnis von Ligand zu Rhodium bei 2 bis 10 liegt.

13. Verfahren nach Anspruch 10, in dem das Molverhältnis von Ligand zu Rhodium bei 2,3 bis 4 liegt.

14. Verfahren nach Anspruch 1, in dem unter ständigen Hydroformylierungsbedingungen in der Reaktionszone das molare Verhältnis von Liganden zu Rh bei 2 bis 5 liegt, das Verhältnis von Rh(mg)/Lösungsmittel (ml) bei 0,07 bis 0,28 ; das Verhältnis von [Olefin-Einspeisung in L(STP)/ Min.]/mg Rh bei 0,03 bis 0,30 liegt ; das Verhältnis von [CO oder $H_2$ Einspeisung in L(STP)/Min.]/mg Rh 0,015 bis 1,5 beträgt, die Temperatur auf 80°C bis 150°C eingestellt wird und der Reaktordruck bei 1655 KPascal bis 1931 KPascal gehalten wird.

15. Verfahren nach einem der Ansprüche 2-9 oder 14, in dem der Ligand 2,2'-Bis(diphenylphosphinomethyl)-1,1'-biphenyl ist.

16. Verfahren nach Anspruch 3, in dem der Ligand der folgenden Formel entspricht :

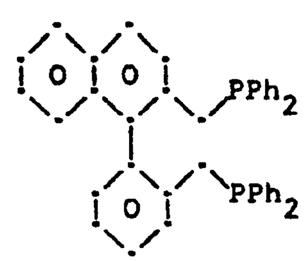

17. Verfahren nach Anspruch 16, in dem das Olefin Propylen ist.

18. Verfahren nach Anspruch 16, in dem das Molverhältnis von Ligand zu Rhodium bei 1 bis 10 liegt.

19. Verfahren nach Anspruch 16, in dem das Molverhältnis von Ligand zu Rhodium bei 2,3 bis 4 liegt.

20. Verfahren nach Anspruch 4, in dem der Ligand der Formel entspricht :

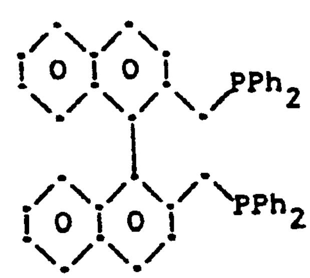

21. Verfahren nach Anspruch 20, in dem das Olefin Propylen ist.

22. Verfahren nach Anspruch 20, in dem das Molverhältnis von Ligand zu Rhodium bei 1 bis 10 liegt.

23. Verfahren nach Anspruch 20, in dem das Molverhältnis von Ligand zu Rhodium bei 2,3 bis 4 liegt.

24. Verbindung der Formel :

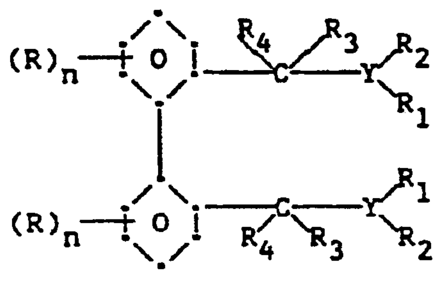

die als Ligand bei der Hydroformylierung und anderen Reaktionen geeignet ist, wobei in der Formel bedeuten :

n gleich 0-4 ;

R jeweils unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br, oder I in der ortho-Position), Alkanoyl, Alkanoyloxy, Alkoxycarbony oder Formyl ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneinander Wasserstoff oder einen $R_1$ Substituenten ;

Y jeweils unabhängig voneinander ausgewählt ist aus den Elementen N, P, As, Sb und Bi und

wobei jede Alkylgruppe oder jeder Rest eine gerade oder verzweigte Kette mit 1-20 Kohlenstoffatomen ist ; jede Arylgruppe 6-10 Ringkohlenstoffatome aufweist und jede cycloaliphatische Gruppe 4-8 Ringkohlenstoffatome enthält,

25. Verbindung nach Anspruch 24, in der n gleich Null ist, $R_1$ und $R_2$ für Alkyl oder Phenyl stehen und $R_3$ und $R_4$ die Bedeutung von Wasserstoff oder Phenyl haben.

26. Verbindung nach Anspruch 24, ausgewählt aus : 2,2′-Bis(diphenylphosphinomethyl)-1,1′-biphenyl; 2,2′-Bis(dibenzylphosphinomethyl)-1,1′-biphenyl ; 2,2′-Bis(phenylbenzylphosphinomethyl)-1,1′-biphenyl ; 2,2′-Bisfdiisobutylphosphinomethyl)-1,1-biphenyl und Mischungen davon.

27. Verbindung nach Anspruch 24, bestehend aus : 2,2′-Bis(diphenylphosphinomethyl)-1,1′-biphenyl.

28. Verbindung der Formel :

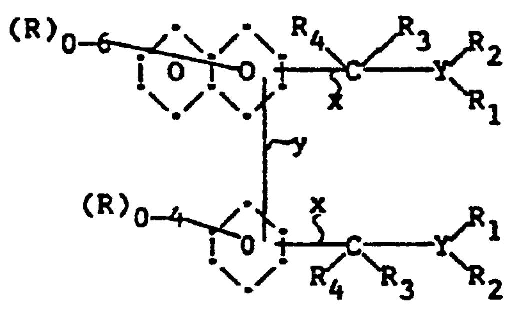

in der bedeuten :

die x-Bindungen und y-Bindungen an benachbarte Kohlenstoffatome der Ringstrukturen gebunden sind ;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in der ortho-Position), Alkanoyl, Alkanoyloxy, Alkoxycarbonyl oder Formyl ;

$R_1$ und $R_2$ jeweils unabhängig voneinander Alkyl, Aryl, Aralkyl, Alkaryl oder eine cycloaliphatische

Gruppe ;

$R_3$ und $R_4$ jeweils unabhängig voneinander Wasserstoff oder ein Substituent $R_1$ ;

jedes Y unabhängig voneinander ausgewählt ist aus den Elementen N, P, As, Sb und Bi und

wobei jede der Alkylgruppen oder jeder der Reste eine gerade oder verzweigte Kette mit 1-20 Kohlenstoffatomen, vorzugsweise 1-8 Kohlenstoffatomen ist, jede Arylgruppe 6-10 Ringkohlenstoffatome und jede cycloaliphatische Gruppe 4-8 Ringkohlenstoffatome aufweist.

29. Verbindung nach Anspruch 28, in der jede Alkylgruppe 1-8 Kohlenstoffatome aufweist.

30. Verbindung nach Anspruch 28, in dem die Ringe unsubstituiert sind, $R_1$ und $R_2$ für Alkyl oder Phenyl stehen und $R_3$ und $R_4$ die Bedeutung von Wasserstoff oder Phenyl haben.

31. Verbindung nach Anspruch 28 der folgenden Struktur :

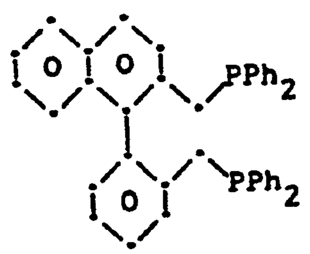

32. Katalysator mit Rhodium, das komplex gebunden ist mit (a) einem Liganden der Formel

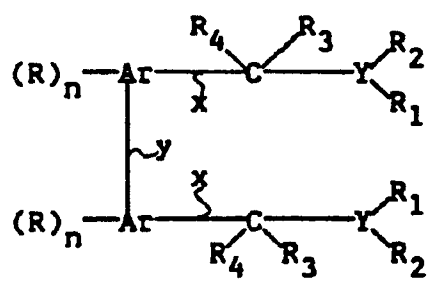

in der bedeuten :

Ar jeweils unabhängig voneinander eine aromatische Ringverbindung mit 6 bis 14 Kohlenstoffatomen, z. B. Phenyl, Naphthyl, Phenanthryl und Anthracenyl ;

die x-Bindungen und y-Bindungen sind an benachbarte Kohlenstoffatome der Ringstruktur gebunden;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in der ortho-Position), Alkanoyl, Alkanoyloxy, Alkoxycarbonyl oder Formyl ;

n eine ganze Zahl von 0-4 ist, wenn Ar für Phenyl steht ; 0-6 ist, wenn Ar für Naphthyl steht ; und 0-8 ist, wenn Ar für Phenanthryl oder Anthracenyl steht ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneiannder Wasserstoff oder ein Substituent $R_1$ ;

wobei jede der Alkylgruppen oder jeder der Reste eine gerade oder verzweigte Kette mit 1-20 Kohlenstoffatomen, vorzugsweise 1-8 Kohlenstoffatomen ist,

jede Arylgruppe 6-10 Ringkohlenstoffatome aufweist ;

jede cycloaliphatische Gruppe 4-8 Ringkohlenstoffatome aufweist und

jedes Y unabhängig voneinander ausgewählt ist aus den Elementen N, P, As, Sb und Bi ;

in einem molaren Verhältnis von Ligand/Rh von 1/1 ;

(b) H in einem atomaren Verhältnis von H/Rh von 1/1 ; und

(c) Kohlenmonoxid in einem molaren Verhältnis von CO/Rh von 2/1.

33. Katalysator nach Anspruch 32, in dem der Ligand der Folgenden Formel entspricht :

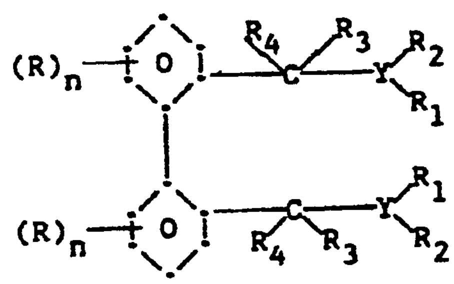

in der bedeuten :

n eine Zahl von 0-4 ;

R jeweils unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in ortho-Position), Alkanoyl, Alkanoyloxy, Alkoxycarbonyl oder Formyl ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneinander Wasserstoff oder einen $R_1$ Substituenten ;

Y jeweils unabhängig voneinander ausgewählt ist aus den Elementen N, P, As, Sb und Bi,

wobei jede Alkylgruppe oder jeder Rest eine gerade oder verzweigte Kette mit 1-20 Kohlenstoffatomen ist ; jede Arylgruppe 6-10 Ringkohlenstoffatome aufweist und jede cycloaliphatische Gruppe 4-8 Ringkohlenstoffatome enthält.

34. Katalysator nach Anspruch 33, in dem der Ligand augesucht ist aus : 2,2′-Bis(diphenylphosphinomethyl)-1,1′-biphenyl ; 2,2′-Bis(dibenzylphosphinomethyl)-1,1′-biphenyl ; 2,2′-Bis(phenylbenzylphosphinomethy1)-1,1′-biphenyl ; 2,2′-Bis(diisobutylphosphinomethyl)-1,1′-biphenyl und Mischungen hiervon.

35. Katalysator nach Anspruch 32, in dem der Ligand der Folgenden Formel entspricht :

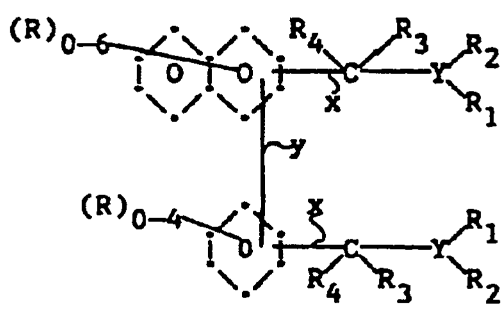

in der bedeuten :

die x-Bindungen und y-Bindungen an benachbarte Kohlenstoffatome der Ringstrukturen gebunden sind ;

R, jeweils, wenn als Substituent vorhanden, unabhängig voneinander Alkyl, Alkoxy, Aryloxy, Aryl, Aralkyl, Alkaryl, Alkoxyalkyl, eine cycloaliphatische Gruppe, Halogen (ausgenommen C1, Br oder I in der ortho-Position), Alkanoyl, Alkanoyloxy, Alkoxycarbonyl oder Formyl ;

$R_1$ und $R_2$ jeweils unabhängig voneinander Alkyl, Aryl, Aralkyl, Alkaryl oder eine cycloaliphatische Gruppe ;

$R_3$ und $R_4$ jeweils unabhängig voneinander Wasserstoff oder ein Substituent $R_1$ ;

wobei jede der Alkylgruppen oder jeder der Reste eine gerade oder verzweigte Kette mit 1-20 Kohlenstoffatomen, jede Arylgruppe 6-10 Ringkohlenstoffatome und jede cycloaliphatische Gruppe 4-8 Ringkohlenstoffatome aufweist und

jedes Y unabhängig voneinander ausgewählt ist aus den Elementen N, P, As, Sb und Bi.

36. Katalysator nach Anspruch 35, in dem die Liganden der folgenden Struktur entsprechen :

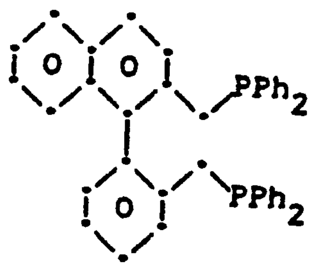

## Revendications

1. Procédé d'hydroformylation dans lequel on met en contact la substance à hydroformyler dans une zone de réaction à une température entre 20°C et 250°C et une pression de 103 kPa (15 psig) à 5517 kPa (800 psig) avec de l'hydrogène, du monoxyde de carbone et un catalyseur comprenant du rhodium sous forme d'un complexe chimique avec un ou plusieurs ligandes de formule :

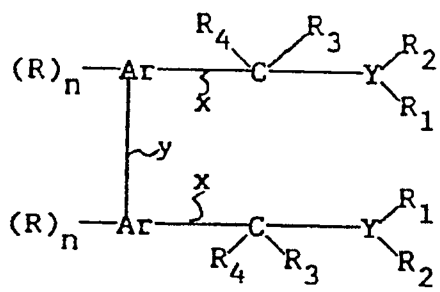

où :

- chaque Ar est choisi indépendamment parmi les composés à cycle aromatique ayant de 6 à 14 atomes de carbone ;
- les liaisons x et y sont rattachées aux atomes de carbone adjacents des structures cycliques ;
- chaque R, lorsqu'il est présent comme substituant, est choisi indépendamment parmi les groupes, alkyle, alkoxy, aryloxy, aryle, aralkyle, alkaryle, alkoxyalkyle, cycloaliphatique, halogène (sauf C1, Br, ou I en position ortho), alkanoyle, alkanoyloxy, alkoxycarbonyle ou formyle ;
- n est un nombre entier, de 0 à 4 quand Ar est phényle, de 0 à 6 quand Ar est naphtyle, et de 0 à 8 quand Ar est phénanthryle ou anthracényle ;
- chaque $R^1$ et $R^2$ est choisi indépendamment parmi les groupes alkyle, aryle, aralkyle, alkaryle ou cycloaliphatique ;
- chaque $R^3$ et $R^4$ est choisi indépendamment parmi l'hydrogène et les substituants $R^1$ ;
- chacun des groupes alkyle précités est à chaîne droite ou ramifiée comprenant de 1 à 20 et de préférence de 1 à 8 atomes de carbone ;
- chaque groupe aryle comprend de 6 à 10 atomes de carbone cycliques ;
- chaque groupe cycloaliphatique comprend de 4 à 8 atomes de carbone cycliques ; et
- chaque Y représente indépendamment l'un des éléments N, P, As, Sb ou Bi,

pendant une durée suffisante pour permettre la réaction des oléfines avec l'hydrogène et le monoxyde de carbone de façon à former des aldéhydes.

2. procédé d'hydroformylation selon la revendication 1, dans lequel le ligande a la formule

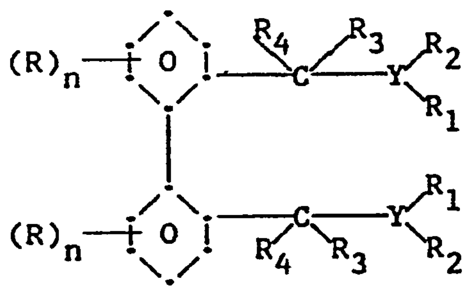

où n est un entier de 0 à 4 ;

– chaque R est choisi indépendamment parmi les groupes alkyle, alkoxy, aryloxy, aryle, aralkyle, alkaryle, alkoxyalkyle, cycloaliphatique, halogène (sauf C1, Br ou I en position ortho), alkanoyle, alkoxycarbonyle, ou formyle ;

– chaque $R^1$ et $R^2$ est choisi indépendamment parmi les groupes alkyle, aryle, aralkyle, alkaryle ou cycloaliphatique ;

– chaque $R^3$ et $R^4$ est choisi indépendamment parmi l'hydrogène et les substituants $R^1$ ;

– où chaque groupe alkyle est à chaîne droite ou ramifiée comprenant de 1 à 20 atomes de carbone, chaque groupe aryle contient de 6 à 10 atomes de carbone cycliques et chaque groupe cycloaliphatique contient de 4 à 8 atomes de carbone cycliques ; et

– chaque Y est indépendamment choisi parmi les éléments N, P, As, Sb et Bi.

3. Procédé d'hydroformylation selon la revendication 1, où le ligande a la formule

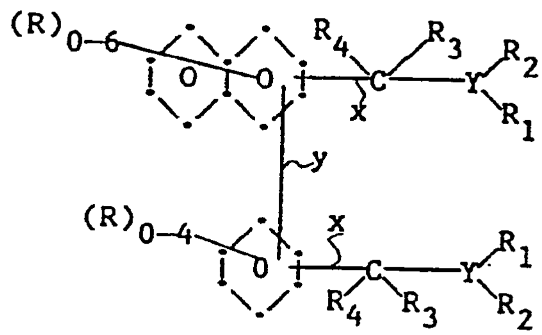

où les liaisons x et y sont rattachées aux atomes de carbone adjacents des structures cycliques ;

– chaque R, lorsqu'il y a un substituant R, est indépendamment choisi parmi les groupes alkyle, alkoxy, aryloxy, aryle, aralkyle, alkaryle, alkoxyalkyle, cycloaliphatique, halogène (excepté C1, Br ou I en position ortho), alkanoyle, alkanoyloxy, alkoxycarbonyle ou formyle ;

– chaque $R^1$ et $R^2$ est indépendamment choisi parmi aryle, alkyle, aralkyle, alkaryle ou cycloaliphatique;

– chaque $R^3$ et $R^4$ est indépendamment choisi parmi l'hydrogène et les substituants $R^1$ ;

– où chacun des groupes alkyle est à chaîne droite ou ramifiée de 1 à 20 atomes de carbone, chacun des groupes aryle contient de 6 à 10 atomes de carbone cycliques et chacun des groupes cycloaliphatique contient de 4 à 8 atomes de carbone cycliques ; et

– chaque Y est choisi indépendamment parmi les éléments N, P, As, Sb et Bi.

4. Procédé d'hydroformylation selon la revendication 1, où le ligande a la formule

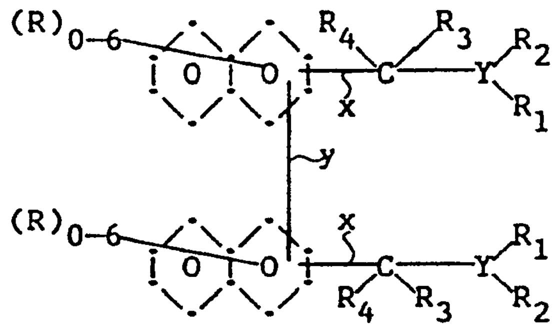

où les liaisons x et y sont rattachées aux atomes de carbone adjacents des structures cycliques ;

– chaque R, lorsqu'il y a un substituant R, est choisi indépendamment parmi les groupes alkyle, alkoxy, aryloxy, aryle, aralkyle, alkaryle, alkoxyalkyle, cycloaliphatique, halogène (excepté C1, Br ou I en position ortho), alkanoyle, alkanoyloxy, alkoxycarbonyle ou formyle ;

– chaque $R^1$ et $R^2$ est choisi indépendamment parmi les groupes alkyle, aryle, aralkyle, alkaryle ou cycloaliphatique ;

– chaque $R^3$ et $R^4$ est choisi indépendamment parmi l'hydrogène et les substituants $R^1$ ;

– où chacun groupe alkyle est à chaîne droite ou ramifiée de 1 à 20 atomes de carbone, chaque groupe aryle contient de 6 à 10 atomes de carbone cycliques et chaque groupe cycloaliphatique contient de 4 à 8 atomes de carbone cycliques ; et

– chaque Y est choisi indépendamment parmi les éléments N, P, As, Sb et Bi.

5. Procédé de la revendication 1 dans lequel les groupes alkyle ont de 1 à 8 atomes de carbone.

6. Procédé d'hydroformylation selon la revendication 1, dans lequel, dans la zone de réaction, on opère à des températures entre environ 80°C et 150°C et à des pressions entre 100 psig et 400 psig environ et le rapport molaire ligande/rhodium est entre 0,5 et 200 environ.

7. Procédé d'hydroformylation selon la revendication 6 dans lequel le rapport molaire de l'hydrogène au monoxyde de carbone est au moins 0,5 et le rapport entre le nombre total de moles d'hydrogène et de monoxyde de carbone présents dans la zone de réaction et le nombre de moles de l'oléfine est compris entre 0,02 et 20.

8. Procédé d'hydroformylation selon la revendication 7, dans lequel la substance à hydroformyler comprend un ou plusieurs des composés suivants : éthylène, propylène, 2-méthylpropylène, 2-butène, 1-butène, 2-méthyl-1-butène, 1-pentène, 1-hexène, 1-heptène, 1-octène, alcool allylique, acétate d'allyle, méthylvinyléther, éthylvinyléther, allyléthyléther, acétate de vinyle, acétate diéthylique de l'acroléine.

9. Procédé d'hydroformylation selon la revendication 1, dans lequel le rhodium est présent dans la zone de réaction à raison de $1 \times 10^{-6}$ à $1 \times 10^{-1}$ mole par mole de l'oléfine présente dans la zone de réaction.

10. Procédé de la revendication 2, dans lequel le ligande est choisi parmi le 2,2'-bis(diphénylphosphinométhyl)-1,1'-biphényle ; le 2,2'-bis(dibenzylphosphinométhyl)-1,1'-biphényle ; le 2,2'-bis(phénylbenzylphosphino-méthyl)-1,1'-biphényle ; le 2,2'-bis(diisobutylphosphinométhyl)-1,1'-biphényle ; ou des mélanges de ces composés.

11. Procédé selon la revendication 10, dans lequel l'oléfine est le propylène.

12. Procédé selon la revendication 10, dans lequel le rapport molaire du ligande au rhodium est compris entre 2 et 10.

13. Procédé selon la revendication 10, dans lequel le rapport molaire du ligande au rhodium est compris entre 2, 3 et 4.

14. Procédé de la revendication 1, dans lequel, aux conditions d'équilibre de l'hydroformylation dans la zone de réaction, le rapport molaire du ligande à Rh est de 2 à 5, le rapport de Rh (mg) au solvant (ml) est de 0,07 à 0,28, le rapport de l'alimentation en oléfine exprimée en L(STP)/min, au Rh (mg) est de 0,03 à 0,30, le rapport de l'alimentation en CO ou $H_2$ exprimée en L(STP)/min au Rh (mg) est de 0,015 à 1,5, la température est maintenue de 80°C à 150°C et la pression du réacteur est maintenue entre 1655 kPa et 1931 kPa (240 psig à 280 psig).

15. Procédé de l'une des revendications 2 à 9 ou 14, où le ligande est le 2,2'-bis(diphénylphosphinométhyl)-1,1'-biphényle.

16. Procédé de la revendication 3, où le ligande a la formule

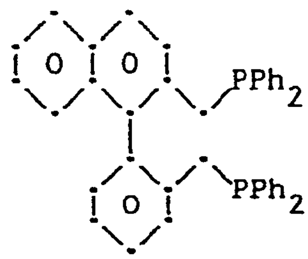

17. Procédé de la revendication 16, où l'oléfine est le propylène.
18. Procédé de la revendication 16, où le rapport molaire du ligande au rhodium est entre 1 et 10.
19. Procédé de la revendication 16, où le rapport molaire du ligande au rhodium est entre 2, 3 et 4.
20. Procédé de la revendication 4, où le ligande a la formule

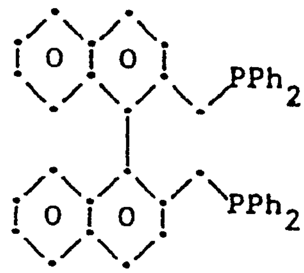

21. Procédé de la revendication 20, où l'oléfine est le propylène.
22. Procédé de la revendication 20, où le rapport molaire du ligande au rhodium est entre 1 et 10.
23. Procédé de la revendication 10, où le rapport molaire du ligande au rhodium est entre 2, 3 et 4.
24. Composé de formule

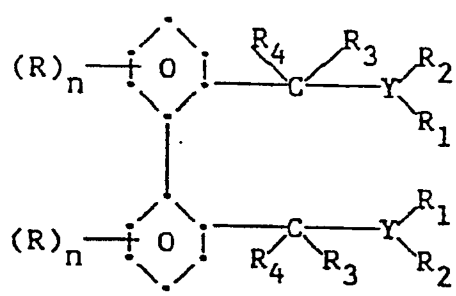

utile comme ligande dans une réaction d'hydroformylation ou dans d'autres réactions, où

– n est un entier de 0 à 4 ;
– chaque R est indépendamment choisi parmi les groupes alkyle, alkoxy, aryloxy, aryle, aralkyle, alkaryle, alkoxyalkyle, cycloaliphatique, halogène (excepté C1, Br, I en position ortho), alkonoyle, alkanoyloxy, alkoxycarbonyle, ou formyle :
– chaque $R^1$ et $R^2$ est indépendamment choisi parmi les groupes alkyle, aryle, aralkyle, alkaryle ou cycloaliphatique ;
– chaque $R^3$ et $R^4$ est indépendamment choisi parmi l'hydrogène et les substituants $R^1$ ;
– chaque Y est choisi indépendamment parmi les éléments N, P, As, Sb et Bi ; et
– chaque groupe alkyle est à chaîne droite ou ramifiée de 1 à 20 atomes de carbone, chaque groupe aryle contient de 6 à 10 atomes de carbone cycliques, chaque groupe cycloaliphatique contient de 4 à 8 atomes de carbone cycliques.

25. Composé selon la revendication 24 où n est 0, $R^1$ et $R^2$ sont alkyle ou phényle, $R^3$ et $R^4$ sont l'hydrogène ou phényle.

26. Composé selon la revendication 24 choisi parmi le 2,2'-bis(diphénylphosphinométhyl)-1,1'-biphényle ; le 2,2'-bis(dibenzylphosphinométhyl)-1,1'-biphényle ; le 2,2'-bis(phénylbenzylphosphinométhyl)-

1,1'-biphényle ; le 2,2'-bis(diisobutylphosphinométhyl)-1,1'-biphényle ; ou des mélanges de ces composés.

27. Composé selon la revendication 24 qui est le 2,2'-bis(diphénylphosphinométhyl)-1,1'-biphényle

28. Composé de formule

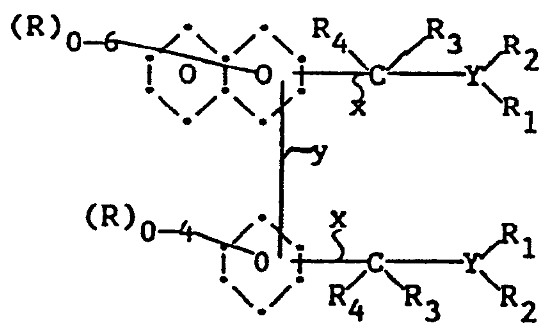

où les liaisons x et y sont rattachées aux atomes de carbone adjacents des structures cycliques ;

- chaque R est indépendamment choisi parmi les groupes alkyle, alkoxy, aryloxy, aryle, aralkyle, alkaryle, alkoxyalkyle, cycloaliphatique, halogène (excepté C1, Br, I en position ortho), alkonoyle, alkanoyloxy, alkoxycarbonyle, ou formyle :
- chaque $R^1$ et $R^2$ est indépendamment choisi parmi alkyle, aryle, aralkyle, alkaryle ou cycloaliphatique;
- chaque $R^3$ et $R^4$ est indépendamment choisi parmi l'hydrogène et les substituants $R^1$ ;
- chaque Y est choisi indépendamment parmi les éléments N, P, As, Sb et Bi ; et
- chaque groupe alkyle est à chaîne droite ou ramifiée de 1 à 20 atomes de carbone, chaque groupe aryle contient de 6 à 10 atomes de carbone cycliques, chaque groupe cycloaliphatique contient de 4 à 8 atomes de carbone cycliques.

29. Composé selon la revendication 28, dans lequel chaque groupe alkyle a de 1 à 8 atomes de carbone.

30. Composé selon la revendication 28, dans lequel les cycles ne sont pas substitués, $R^1$ et $R^2$ sont alkyle ou phényle, $R^3$ et $R^4$ sont l'hydrogène ou phényle.

31. Composé selon la revendication 28, de formule

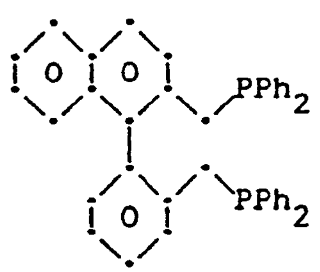

32. Catalyseur comprenant du rhodium complexé avec (a) un ligande de formule

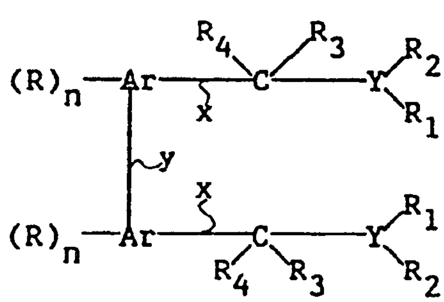

où

- chaque Ar est indépendamment choisi parmi les composés à cycle aromatique ayant de 6 à 14 atomes de carbone, par exemple phényle, naphtyle, phénantryle et anthracényle ;

– les liaisons x et y sont rattachées aux atomes de carbone adjacents des structures cycliques ;
– chaque R, quand il y a un substituant R est indépendamment choisi parmi les alkyle, alkoxy, aryloxy, aryle, aralkyle, alkaryle, alkoxyalkyle, cycloaliphatique, halogène (excepté C1, Br, I en position ortho), alkanoyle, alkanoyloxy, alkoxycarbonyle ou formyle :
– n est un nombre entier de 0 à 4 quand Ar est phényle ; de 0 à 6 quand Ar est naphtyle et de 0 à 8 quand Ar est phénanthryle ou anthracényle ;
– chaque $R^1$ et $R^2$ est indépendamment choisi parmi les groupes alkyle, aryle, aralkyle, alkaryle ou cycloaliphatique ;
– chaque $R^3$ et $R^4$ est indépendamment choisi parmi l'hydrogène et les substituants $R^1$ ;
– chaque groupe alkyle est à chaîne droite ou ramifiée de 1 à 20 atomes de carbone, de préférence de 1 à 8 atomes de carbone ;
– chaque groupe aryle contient de 6 à 10 atomes de carbone cycliques ;
– chaque groupe cycloaliphatique contient de 4 à 8 atomes de carbone cycliques ; et
– chaque Y indépendamment choisi parmi les éléments N, P, As, Bi, et Sb ;
dans un rapport molaire ligande/Rh de 1/1 ;
(b) H dans un rapport atomique H/Rh de 1/1 ; et
(c) du monoxyde de carbone dans un rapport molaire CO/Rh de 2/1.

33. Catalyseur selon la revendication 32, dans lequel le ligande a la formule :

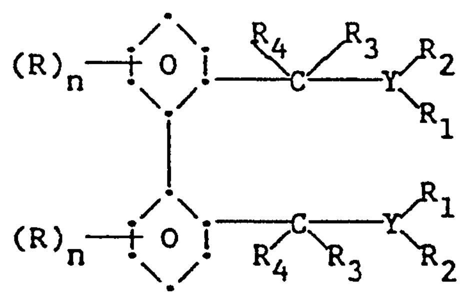

où n est un nombre entier compris entre 0 et 4 ;
– chaque R est indépendamment choisi parmi les groupes alkyle, alkoxy, aryloxy, aryle, aralkyle, alkaryle, alkoxyalkyle, cycloaliphatique, halogène (excepté C1, Br ou I en position ortho), alkanoyle, alkanoyloxy, alkoxycarbonyle, ou formyle ;
– chaque $R^1$ et $R^2$ est indépendamment choisi parmi les groupes alkyle, aryle, aralkyle, alkaryle, ou cycloaliphatique ;
– chaque $R^3$ et $R^4$ est indépendamment choisi parmi l'hydrogène et les substituants $R^1$ ;
– chaque Y est indépendamment choisi parmi les éléments N, P, As, Sb et Bi ; et
– chaque groupe alkyle est à chaîne droite ou ramifiée de 1 à 20 atomes de carbone, chaque groupe aryle contient de 6 à 10 atomes de carbone cycliques, chaque groupe cycloaliphatique contient de 4 à 8 atomes de carbone cycliques.

34. Catalyseur selon la revendication 33, où le ligande est choisi parmi 2,2'-bis(diphénylphosphinométhyl)-1,1'-biphényle ; le 2,2'-bis(dibenzylphosphinométhyl)-1,1'-biphényle ; le 2,2'-bis(phénylbenzyl phosphinométhyl)-1,1'-biphényle ; le 2,2'-bis-(diisobutylphosphinométhyl)-1,1''-biphényle ; ou des mélanges de ces composés.

35. Catalyseur selon la revendication 32, où le ligande a la formule

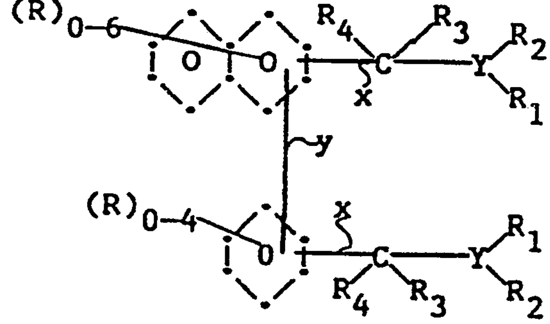

où les liaisons x et y sont rattachées aux atomes de carbone adjacents des structures cycliques

- chaque R, quand il y a un substituant R est indépendamment choisi parmi les groupes aryle, alkoxy, aryloxy, aryle, aralkyle, alkaryle, alkoxyalkyle, cycloaliphatique, halogène (excepté C1, Br, I en position ortho), alkanoyle, alkanoyloxy, alkoxycarbonyle ou formyle :
- chaque $R^1$ et $R^2$ est indépendamment choisi parmi les groupes alkyle, aryle, aralkyle, alkaryle ou cycloaliphatique ;
- chaque $R^3$ et $R^4$ est indépendamment choisi parmi l'hydrogène et les substituants $R^1$ ;
- chaque groupe alkyle est à chaîne droite ou ramifiée de 1 à 20 atomes de carbone ; chaque groupe aryle contient de 6 à 10 atomes de carbone cycliques ; chaque groupe cycloaliphatique contient de 4 à 8 atomes de carbone cycliques ; et
- chaque Y indépendamment choisi parmi les éléments N, P, As, Bi, et Sb.

36. Catalyseur selon la revendication 35, où le ligande a la structure